(19) Europäisches Patentamt
European Patent Office
Office européen des brevets

(11) **EP 4 722 332 A1**

(12) **EUROPEAN PATENT APPLICATION**
published in accordance with Art. 153(4) EPC

(43) Date of publication:
**08.04.2026 Bulletin 2026/15**

(21) Application number: **24823479.1**

(22) Date of filing: **14.06.2024**

(51) International Patent Classification (IPC):
*C12M 1/00* (2006.01)     *C12M 1/34* (2006.01)
*C12N 5/00* (2006.01)

(52) Cooperative Patent Classification (CPC):
**C12M 1/00; C12M 1/34; C12N 5/00**

(86) International application number:
**PCT/JP2024/021739**

(87) International publication number:
**WO 2024/257866 (19.12.2024 Gazette 2024/51)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **16.06.2023 JP 2023098863**

(71) Applicant: **Terumo Kabushiki Kaisha
Tokyo 151-0072 (JP)**

(72) Inventor: **YAMAZAKI, Nozomu
Ashigarakami-gun, Kanagawa 259-0151 (JP)**

(74) Representative: **TBK
Bavariaring 4-6
80336 München (DE)**

(54) **SIMULATION DEVICE, SIMULATION SYSTEM, AND SIMULATION METHOD**

(57)     A simulation apparatus (14) includes a simulation execution unit (142) that executes a simulation regarding a change in concentration of protein in an inner circulation path along with cell proliferation under a predetermined culture condition at least using a supply speed that is a speed at which the concentration of protein changes in the inner circulation path by supply of a culture medium to the inner circulation path (first circulation flow path (58)), an exchange speed that is a speed at which the concentration of protein changes in the inner circulation path by inflow and outflow of the culture medium between an inside of an inner pore and an outside of the inner pore via a hollow fiber membrane (40), and a deterioration speed that is a speed at which the concentration of protein changes in the inner circulation path due to deterioration of protein.

FIG. 3

EP 4 722 332 A1

**Description**

Technical Field

The present invention relates to a simulation apparatus, a simulation system, and a simulation method for simulating cell proliferation by a cell culture apparatus.

Background Art

JP 2020-171241 A discloses a cell culture apparatus. The cell culture apparatus includes a bioreactor, a supply unit, a recovery unit, and a plurality of flow paths. Some flow paths form a circulation path with the bioreactor. The supply unit supplies a cell-containing solution and a culture medium (culture solution) to the bioreactor. The bioreactor cultures cells. During cell culture, a part of the culture medium (first culture medium) circulates in the circulation path. During the cell culture, a part of the culture medium (second culture medium) is discharged as a waste liquid. The cultured cells are recovered by the recovery unit.

Citation List

Patent Literature

Patent Literature 1: JP 2020-171241 A

Summary of Invention

Technical Problem

Supply of nutrients (glucose, glutamine, various amino acids and the like), supply of gases (oxygen, carbon dioxide and the like), and discharge of waste products (lactic acid, ammonia and the like) are important for cell culture. Furthermore, protein supply is also important for cell culture. Therefore, bovine serum (including albumin and growth factors), growth factors, cytokines and the like are added to the culture medium.

When protein supplied to the cell is insufficient, the cell does not proliferate. In contrast, when the amount of protein to be supplied to the cell is large, the cell proliferation is rather inhibited. Furthermore, a unit price of some proteins (growth factors, cytokines and the like) is high. For these reasons, it is desirable to appropriately control the amount of protein to be supplied to cells.

Solution to Problem

An object of the present invention is to solve the above-described problem.

(1) A first invention is a simulation apparatus that executes a simulation regarding cell proliferation in a cell culture apparatus that supplies a culture medium containing protein to an inner circulation path that is a circulation path including an inner pore of a cylindrical hollow fiber membrane to culture cells in the inner pore, the simulation apparatus including a simulation execution unit that executes the simulation regarding a change in concentration of the protein in the inner circulation path along with the cell proliferation under a predetermined culture condition at least using a supply speed that is a speed at which the concentration of the protein changes in the inner circulation path by supply of the culture medium to the inner circulation path, an exchange speed that is a speed at which the concentration of the protein changes in the inner circulation path by inflow and outflow of the culture medium between an inside of the inner pore and an outside of the inner pore via the hollow fiber membrane, and a deterioration speed that is a speed at which the concentration of the protein changes in the inner circulation path due to deterioration of the protein.

From a relationship between a molecular weight cutoff of the hollow fiber membrane and a size of protein, a behavior of the protein diffusing into and out of the cylindrical hollow fiber membrane differs for each type of protein. In the first invention, the simulation is executed using not only the supply speed and the deterioration speed but also the exchange speed. Therefore, according to the first invention, the accuracy of the simulation of the change in concentration of protein can be improved. The user can appropriately control an amount of protein by reflecting the simulation result in the actual culture.

(2) The simulation apparatus according to the above-described item (1) may further include a display control unit that allows a display unit to display information according to the concentration change obtained by the simulation.

(3) In the simulation apparatus according to the above-described item (1) or (2), the simulation execution unit may execute the simulation regarding the change in concentration of the protein in the inner circulation path by further using a consumption speed that is a speed at which the concentration of the protein changes in the inner circulation path by consumption of the protein by the cells.

According to the configuration of the above-described item (3), the accuracy of the simulation of the change in concentration of protein can be further improved.

(4) In the simulation apparatus according to any one of the above-described items (1) to (3), in a case where a part of the culture medium is discarded via the inner circulation path during cell culture, the simulation execution unit may execute the simulation regarding the change in concentration of the protein in the inner circulation path by further using a discard speed that is a speed at which the concentration of the protein changes in the inner circulation path by discarding a part of the culture medium via the inner circulation path.

According to the configuration of the above-described item (4), the accuracy of the simulation of the change in concentration of protein can be further improved.

(5) In the simulation apparatus according to any one of the above-described items (1) to (4), the simulation execution unit may execute the simulation regarding the change in concentration of the protein in the inner circulation path by further using an adsorption rate that is a ratio of the protein that cannot be consumed by the cells as the protein is adsorbed, aggregated, and deposited on at least one of an inside of the inner circulation path and an inside of an outer circulation path that is a circulation path including a region between the hollow fiber membrane and a housing that stores the hollow fiber membrane.

According to the configuration of the above-described item (5), the accuracy of the simulation of the change in concentration of protein can be further improved.

(6) In the simulation apparatus according to any one of the above-described items (1) to (5), the cell culture apparatus is capable of supplying a culture medium not containing the protein to an outer circulation path that is a circulation path including a region between the hollow fiber membrane and a housing that stores the hollow fiber membrane.

(7) In the simulation apparatus according to any one of the above-described items (1) to (6), in a case of culturing adherent cells, the cell culture apparatus may discard the culture medium via a waste liquid flow path connected to the inner circulation path.

(8) In the simulation apparatus according to the above-described item (6), in a case of culturing floating cells, the cell culture apparatus may discard the culture medium via a waste liquid flow path connected to the outer circulation path.

(9) In the simulation apparatus according to any one of the above-described items (1) to (8), the cell culture apparatus includes an inside of the inner circulation path and an outer circulation path that is a circulation path including a region between the hollow fiber membrane and a housing that stores the hollow fiber membrane, in a case where a part of the culture medium is discarded via the outer circulation path during cell culture, the simulation execution unit may execute the simulation regarding the change in concentration of the protein in the outer circulation path along with the cell proliferation under the culture condition by using at least a discard speed that is a speed at which the concentration of the protein changes in the outer circulation path by discarding a part of the culture medium via the outer circulation path, the exchange speed, and the deterioration speed.

(10) A second invention is a simulation system that executes a simulation regarding cell proliferation in a cell culture apparatus that supplies a culture medium containing protein to an inner circulation path that is a circulation path including an inner pore of a cylindrical hollow fiber membrane to culture cells in the inner pore, the simulation system including a simulation execution unit that executes the simulation regarding a change in concentration of the protein in the inner circulation path along with the cell proliferation under a predetermined culture condition at least using a supply speed that is a speed at which the concentration of the protein changes in the inner circulation path by supply of the culture medium to the inner circulation path, an exchange speed that is a speed at which the concentration of the protein changes in the inner circulation path by inflow and outflow of the culture medium between an inside of the inner pore and an outside of the inner pore via the hollow fiber membrane, and a deterioration speed that is a speed at which the concentration of the protein changes in the inner circulation path due to deterioration of the protein; and a display control unit that allows a display unit to display information according to the change in concentration obtained by the simulation.

In the second invention, the simulation is executed using not only the supply speed and the deterioration speed but also the exchange speed. Therefore, according to the second invention, the accuracy of the simulation of the change in concentration of protein can be improved. The user can appropriately control an amount of protein by reflecting the simulation result in the actual culture.

(11) A third invention is a simulation method that executes a simulation regarding cell proliferation in a cell culture apparatus that supplies a culture medium containing protein to an inner circulation path that is a circulation path including an inner pore of a cylindrical hollow fiber membrane to culture cells in the inner pore, the simulation method including a simulation step of executing the simulation regarding a change in concentration of the protein in the inner circulation path along with the cell proliferation under a predetermined culture condition at least using a supply speed that is a speed at which the concentration of the protein changes in the inner circulation path by supply of the culture medium to the inner circulation path, an exchange speed that is a speed at which the concentration of the protein changes in the inner circulation path by inflow and outflow of the culture medium between an inside of the inner pore and an outside of the inner pore via the hollow fiber membrane, and a deterioration speed that is a speed at which the concentration of the protein changes in the inner circulation path due to deterioration of the protein.

In the third invention, the simulation is executed using not only the supply speed and the deterioration speed but also the exchange speed. Therefore, according to the third invention, the accuracy of the simulation of the change in concentration of protein can be improved. The user can appropriately control an amount of protein by reflecting the simulation result in the actual culture.

(12) The simulation method according to the above-described item (11) can further include a display step of allowing a display unit to display information according to the change in concentration obtained by the simulation.

(13) In the simulation method according to the above-described item (11) or (12), at the simulation step, the simulation regarding the change in concentration of the protein in the inner circulation path can be executed by further using a consumption speed that is a speed at which the concentration of the protein changes in the inner circulation path by consumption of the protein by the cells.

According to the configuration of the above-described item (13), the accuracy of the simulation of the change in concentration of protein can be further improved.

(14) In the simulation method according to any one of the above-described items (11) to (13), in a case where a part of the culture medium is discarded via the inner circulation path during cell culture, at the simulation step, the simulation regarding the change in concentration of the protein in the inner circulation path can be executed by further using a discard speed that is a speed at which the concentration of the protein changes in the inner circulation path by discarding a part of the culture medium via the inner circulation path.

According to the configuration of the above-described item (14), the accuracy of the simulation of the change in concentration of protein can be further improved.

(15) In the simulation method according to any one of the above-described items (11) to (14), at the simulation step, the simulation regarding the change in concentration of the protein in the inner circulation path may be executed by further using an adsorption rate that is a ratio of the protein that cannot be consumed by the cells as the protein is adsorbed, aggregated, and deposited on at least one of an inside of the inner circulation path and an inside of an outer circulation path that is a circulation path including a region between the hollow fiber membrane and a housing that stores the hollow fiber membrane.

According to the configuration of the above-described item (15), the accuracy of the simulation of the change in concentration of protein can be further improved.

(16) In the simulation method according to any one of the above-described items (11) to (15), the cell culture apparatus may be capable of supplying a culture medium not containing the protein to an outer circulation path that is a circulation path including a region between the hollow fiber membrane and a housing that stores the hollow fiber membrane.

(17) In the simulation method according to any one of the above-described items (11) to (16), in a case of culturing adherent cells, the cell culture apparatus may discard the culture medium via a waste liquid flow path connected to the inner circulation path.

(18) In the simulation method according to the above-described item (16), in a case of culturing floating cells, the cell culture apparatus may discard the culture medium via a waste liquid flow path connected to the outer circulation path.

(19) In the simulation method according to any one of the above-described items (11) to (18), the cell culture apparatus includes an inside of the inner circulation path and an outer circulation path that is a circulation path including a region between the hollow fiber membrane and a housing that stores the hollow fiber membrane, in a case where a part of the culture medium is discarded via the outer circulation path during cell culture, at the simulation step, the simulation regarding the change in concentration of the protein in the outer circulation path along with the cell proliferation under the culture condition can be executed by using at least a discard speed that is a speed at which the concentration of the protein changes in the outer circulation path by discarding a part of the culture medium via the outer circulation path, the exchange speed, and the deterioration speed.

According to the present invention, the amount of protein can be appropriately controlled.

Brief Description of Drawings

Fig. 1 is a diagram illustrating a configuration of a cell culture system.

Fig. 2 is a diagram illustrating a configuration of a control unit of a cell culture apparatus.

Fig. 3 is a diagram illustrating a configuration of a simulation apparatus.

Fig. 4 is a table illustrating parameter information.

Fig. 5 is a diagram illustrating an input screen displayed on a display unit.

Fig. 6 is a diagram illustrating a proliferation data screen displayed on the display unit.

Fig. 7 is a diagram illustrating a feedback condition screen displayed on the display unit.

Fig. 8 is a diagram illustrating a results screen displayed on the display unit.

Fig. 9 is a diagram illustrating a result screen displayed on the display unit.

Fig. 10 is a flowchart illustrating a flow of a cell culture method performed using the cell culture system.

Fig. 11 is a diagram illustrating an operation of the cell culture apparatus at the time of cell culture in a first culture form.

Fig. 12 is a diagram illustrating an operation of the cell culture apparatus at the time of cell collection in the first culture form.

Fig. 13 is a diagram illustrating an operation of the cell culture apparatus at the time of cell culture in a second culture form.

Fig. 14 is a diagram illustrating an operation of the cell culture apparatus at the time of cell culture in a third culture form.

Fig. 15 is a diagram illustrating an operation of the cell culture apparatus at the time of cell culture in a fourth culture form.

Fig. 16 is a flowchart illustrating a flow of the cell culture performed using the cell culture apparatus.

Fig. 17 is a diagram illustrating an operation of the cell culture apparatus at the time of cell culture.

Fig. 18 is a diagram illustrating an operation of the cell culture apparatus at the time of cell detachment.

Fig. 19 is a diagram illustrating an operation of the cell culture apparatus at the time of cell recovery.

Fig. 20 is a diagram illustrating a configuration of a simulation system.

Fig. 21 is a table illustrating a specific example of numerical values of parameter information.

Fig. 22 is a table illustrating a culture condition.

Fig. 23 is a table illustrating a culture condition.

Fig. 24 is a table illustrating a culture condition.

Fig. 25 is a graph illustrating transition of a concentration of protein in a first circulation flow path.

Fig. 26 is a graph illustrating transition of the concentration of protein in the first circulation flow path.

Fig. 27 is a graph illustrating transition of the concentration of protein in the first circulation flow path.

Fig. 28 is a table illustrating a culture condition.

Fig. 29 is a graph illustrating transition of the concentration of protein in the first circulation flow path.

Description of Embodiments

In cell culture using a hollow fiber membrane, due to a relationship between a fractionated size of the hollow fiber membrane and a molecular weight of a protein, a protein smaller than the fractionated size flows out of the hollow fiber membrane during a culture period. Therefore, it has been difficult to accurately grasp a protein concentration.

Examples of cells to be cultured include adherent cells that grow using the hollow fiber membrane as a scaffold, and floating cells that float and grow in the hollow fiber membrane. In order to culture cells, a cell culture form according to a cell type is required. In order to accurately grasp the protein concentration, a method of predicting a change in protein concentration according to the cell culture form is required.

Furthermore, it is necessary to change various cell culture forms according to a cell proliferation state, for example, to maintain a nutrient source in a cell proliferation period in a culture initial stage and enrich the nutrient source in culture middle to later stages. It is difficult to predict the change in protein concentration during a series of culture periods.

The embodiment described below makes it possible to accurately grasp the protein concentration. The embodiment described below makes it possible to predict the change in protein concentration according to the cell culture form. The embodiment described below makes it possible to predict the change in protein concentration in a series of culture periods.

[1 Configuration of Cell Culture System 10]

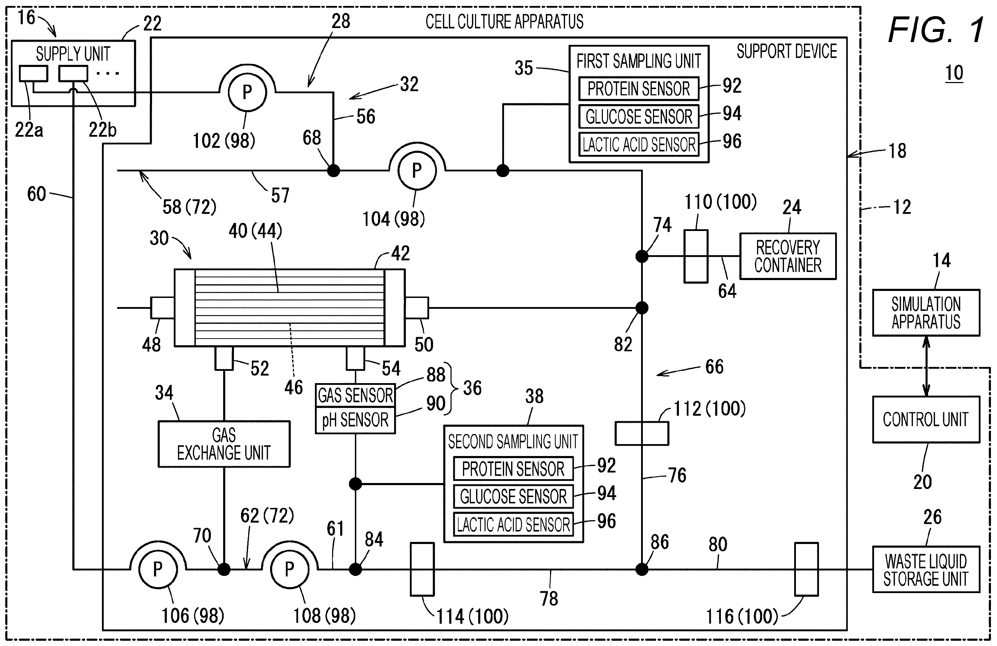

Fig. 1 is a diagram illustrating a configuration of a cell culture system 10. The cell culture system 10 cultures (proliferates) cells separated from biological tissue in a culture medium. The cells used in the cell culture system 10 may be mammalian cells or cells derived from a mammal. The cells used in the cell culture system 10 may be adherent cells or floating cells. Examples of the adherent cells include, for example, human embryonic kidney cells (HEK 293 cells), embryonic stem cells (ES cells), induced pluripotent stem cells (iPS cells), mesenchymal stem cells, fibroblast cells, endothelial cells, neural stem cells and the like. Examples of the floating cells include Jurkat cells (human cellular leukemia-derived cells), T cells, regulatory T cells, tumor-infiltrating lymphocytes, CAR-T cells, CD34 positive cells and the like. The cells used in the cell culture system 10 are not limited to those described above.

The cell culture system 10 includes a cell culture apparatus 12 and a simulation apparatus 14. The cell culture apparatus 12 includes a cell culture circuit 16, a support device 18, and a control unit 20. A liquid flows in the cell culture circuit 16. The liquid includes at least one of a cell fluid, a culture medium, a cleaning liquid, and a detaching liquid. The cell fluid is a solution containing cells. The culture medium is a culture solution for proliferating the cells. The culture medium is selected according to the cells to be cultured. In the present embodiment, two kinds of culture media, which are a basal medium and a complete medium, are used. As the basal medium, for example, a minimum essential medium (MEM) is used. As the complete medium, a basal medium (for example, MEM) containing protein is used. As the protein, albumin, growth factors, cytokines and the like are used. For example, bovine serum containing albumin, growth factors and the like is added to the basal medium. The cleaning liquid cleans the inside of the cell culture circuit 16. As the cleaning liquid, for example, water, a buffer solution, physiological saline or the like is used. Examples of the buffer solution include phosphate buffered saline (PBS), tris-buffered saline (TBS) and the like. The detaching liquid detaches cells from a bioreactor 30 to be described later of the cell culture circuit 16. As the detaching liquid, for example, trypsin, an EDTA solution or the like is used. The culture medium, the cleaning liquid, and the detaching liquid are not limited to the liquid described above.

[1-1 Cell Culture Apparatus 12]

[1-1-1 Cell Culture Circuit 16]

The cell culture circuit 16 is discarded after a single use. In other words, the cell culture circuit 16 is discarded every time a predetermined number of cells are cultured. That is, the cell culture circuit 16 is a disposable product. The cell culture circuit 16 includes a supply unit 22, a recovery container 24, a waste liquid storage unit 26, and a culture main body 28.

The supply unit 22 supplies the liquid such as the cell fluid, the culture medium, the cleaning liquid, and the detaching liquid to the culture main body 28. The supply unit 22 includes a first supply unit 22a and a second supply unit 22b. The recovery container 24 recovers the cells cultured in the culture main body 28. The waste liquid storage unit 26 stores a waste liquid generated in the culture main body 28. Each of the recovery container 24 and the waste liquid storage unit 26 is, for example, a medical bag obtained by shaping a soft resin material into a bag shape. Each of the recovery container 24 and the waste liquid storage unit 26 may be a tank and the like formed of a hard material.

The culture main body 28 includes the bioreactor 30, a flow path 32, a gas exchange unit 34, a first sampling unit 35, a sensor unit 36, and a second sampling unit 38.

The bioreactor 30 includes a plurality of hollow fiber membranes 40 and a cylindrical housing 42. The plurality of hollow fiber membranes 40 is stored in the housing 42. The hollow fiber membrane 40 has a cylindrical shape. That is, the hollow fiber membrane 40 includes a pore (inner pore) penetrating from a first end to a second end. The first end of each hollow fiber membrane 40 is fixed to a first end of the housing 42. The second end of each hollow fiber membrane 40 is fixed to a second end of the housing 42. The hollow fiber membrane 40 is formed of, for example, a polymer material.

The bioreactor 30 includes a first region 44 and a second region 46. The first region 44 is the inner pore of the plurality of hollow fiber membranes 40. The first region 44 is a region where cells are present in the culture medium. This region is also referred to as a culture region. The second region 46 is a space between an inner peripheral surface of the housing 42 and outer peripheral surfaces of the plurality of hollow fiber membranes 40. The second region 46 is a non-culture region of an inner region of the bioreactor 30. Each hollow fiber membrane 40 includes a plurality of pores not illustrated. The first region 44 and the second region 46 communicate with each other via the plurality of pores of each hollow fiber membrane 40. A diameter of each pore is a size that allows passage of low molecules (for example, water, ions, oxygen, lactate and the like) while blocking passage of macromolecules (cells and the like). The diameter of each pore is set to, for example, 0.005 [μm] or larger and 10 [μm] or smaller.

A first inlet port 48, a first outlet port 50, a second inlet port 52, and a second outlet port 54 are attached to the housing 42. The first inlet port 48 is attached to the first end of the housing 42. The first inlet port 48 communicates with the first region 44 via an inlet located at the first end of the plurality of hollow fiber membranes 40. The first outlet port 50 is attached to the second end of the housing 42. The first outlet port 50 communicates with the first region 44 via an outlet located at the second end of the plurality of hollow fiber membranes 40.

The second inlet port 52 and the second outlet port 54 are attached to an outer peripheral surface of the housing 42. The second inlet port 52 is located between the center of the housing 42 and the first inlet port 48 in a longitudinal direction of the housing 42. The second outlet port 54 is located between the center of the housing 42 and the first outlet port 50 in the longitudinal direction of the housing 42. Each of the second inlet port 52 and the second outlet port 54 communicates with the second region 46.

The flow path 32 includes a plurality of tubes through which a liquid flows. Each tube is formed of a soft resin material. The flow path 32 includes a first supply flow path 56, a first communication flow path 57, a second supply flow path 60, a second communication flow path 61, a recovery flow path 64, and a waste liquid flow path 66.

One end of the first supply flow path 56 is connected to the first supply unit 22a. The first supply unit 22a supplies the cell fluid, the complete medium, the basal medium, the cleaning liquid, and the detaching liquid one by one to the first supply flow path 56 at a predetermined timing. The other end of the first supply flow path 56 is connected to a first junction 68 of the first communication flow path 57.

The first junction 68 is located between a first end and a second end of the first communication flow path 57. The first end of the first communication flow path is connected to the first inlet port 48. That is, the first communication flow path 57 communicates with the first region 44 in the inner pore of each hollow fiber membrane 40 via the first inlet port 48. The second end of the first communication flow path 57 is connected to the first outlet port 50. That is, the first communication flow path 57 communicates with the first region 44 in the inner pore of each hollow fiber membrane 40 via the first outlet port 50. A closed circuit formed by the first communication flow path 57 and the first region 44 is referred to as a first circulation flow path (inner circulation path) 58.

One end of the second supply flow path 60 is connected to the second supply unit 22b. The second supply unit 22b supplies the basal medium and the cleaning liquid one by one to the second supply flow path 60 at a predetermined timing. The other end of the second supply flow path 60 is connected to a second junction 70 of the second communication flow path 61.

The second junction 70 is located between a first end and a second end of the second communication flow path 61. The first end of the second communication flow path 61 is connected to the second inlet port 52. That is, the second communication flow path 61 communicates with the second region 46 outside the inner pore of each hollow fiber membrane 40 via the second inlet port 52. The second end of the second communication flow path 61 is connected to the second outlet port 54. That is, the second communication flow path 61 communicates with the second region 46 outside the inner pore of each hollow fiber membrane 40 via the second outlet port 54. A closed circuit formed by the second communication flow path 61 and the second region 46 is referred to as a second circulation flow path (outer circulation path) 62. Hereinafter, the first circulation flow path 58 and the second circulation flow path 62 might be collectively referred to as a "circulation flow path 72".

The recovery flow path 64 extends from the first circulation flow path 58. One end of the recovery flow path 64 is connected to a recovery branch 74 of the first circulation flow path 58. The recovery branch 74 is located between the first junction 68 and the first outlet port 50 in the first circulation flow path 58. The other end of the recovery flow path 64 is connected to the recovery container 24.

A liquid to be discarded from the circulation flow path 72 flows through the waste liquid flow path 66. The waste liquid flow path 66 includes a first waste liquid flow path 76, a second waste liquid flow path 78, and a third waste liquid flow path 80. The first waste liquid flow path 76 extends from the first circulation flow path 58. One end of the first waste liquid flow path 76 is connected to the first branch 82 of the first circulation flow path 58. The first branch 82 is located between the first outlet port 50 and the recovery branch 74 of the first circulation flow path 58. The second waste liquid flow path 78 extends from the second circulation flow path 62. One end of the second waste liquid flow path 78 is connected to the second branch 84 of the second circulation flow path 62. The second branch 84 is located between the second junction 70 and the second outlet port 54 of the second circulation flow path 62. The other end of the first waste liquid flow path 76 and the other end of the second waste liquid flow path 78 are connected to each other at an intermediate junction 86. One end of the third waste liquid flow path 80 is connected to the first waste liquid flow path 76 and the second waste liquid flow path 78 at the intermediate junction 86. The other end of the third waste liquid flow path 80 is connected to the waste liquid storage unit 26.

The gas exchange unit 34 is attached between the second junction 70 and the second inlet port 52 of the second circulation flow path 62. The gas exchange unit 34 allows a gas of a predetermined component to pass through a liquid (basal medium) flowing through the second circulation flow path 62. The gas used in the gas exchange unit 34 contains, for example, components similar to those of air in nature. In other words, the gas contains nitrogen, oxygen, and carbon dioxide. Specifically, the gas contains, for example, 75% nitrogen, 20% oxygen, and 5% carbon dioxide in volume ratio.

The first sampling unit 35 is connected to the first circulation flow path 58. The first sampling unit 35 extracts a part of a liquid (complete medium) that flows through the first circulation flow path 58, and measures components contained in the liquid. For example, the first sampling unit 35 aseptically collects a tube fragment containing an internal liquid from a tube having a sufficient length using a sterile joining device. The first sampling unit 35 includes a protein sensor 92, a glucose sensor 94, and a lactic acid sensor 96.

The sensor unit 36 is attached between the second outlet port 54 and the second branch 84 of the second circulation flow path 62. The sensor unit 36 includes a gas sensor 88 and a pH sensor 90. The gas sensor 88 measures a gas concentration of a liquid flowing through the second circulation flow path 62. Specifically, the gas sensor 88 includes an oxygen sensor and a carbon dioxide sensor. The oxygen sensor measures an oxygen concentration of the liquid flowing through the second circulation flow path 62. The carbon dioxide sensor measures a carbon dioxide concentration of the liquid flowing through the second circulation flow path 62. The pH sensor 90 measures pH (hydrogen ion index) of the liquid flowing through the second circulation flow path 62. Each of the gas sensor 88 and the pH sensor 90 outputs a measurement result to the control unit 20.

The second sampling unit 38 is connected to a portion between the second outlet port 54 and the second branch 84 of the second circulation flow path 62. The second sampling unit 38 extracts a part of the liquid flowing through the second circulation flow path 62, and measures a component contained in the liquid. The second sampling unit 38 includes a protein sensor 92, a glucose sensor 94, and a lactic acid sensor 96 similarly to the first sampling unit 35.

The protein sensor 92 measures a protein concentration of the liquid extracted from the second circulation flow path 62. The glucose sensor 94 measures a glucose concentration of the liquid extracted from the second circulation flow path 62. The lactic acid sensor 96 measures a lactic acid concentration of the liquid extracted from the second circulation flow path 62. Each of the protein sensor 92, the glucose sensor 94, and the lactic acid sensor 96 outputs a measurement result to the control unit 20.

[1-1-2 Support Device 18]

The cell culture circuit 16 described above is set in the support device 18. The support device 18 includes a cassette that supports the cell culture circuit 16. The support device 18 is a reusable product that can be used a plurality of times.

The support device 18 includes a plurality of pumps 98 and a plurality of clamps 100. Each of the plurality of pumps 98 applies a flow force to the liquid in the flow path 32 by squeezing a wall of the flow path 32. Each of the plurality of pumps 98 includes a pressing member (not illustrated). The pressing member includes, for example, a rotating member and a plurality of pressing rollers. The plurality of pressing rollers is attached to an outer peripheral portion of the rotating member. The plurality of pressing rollers is arranged at intervals in a circumferential direction of the rotating member. Each pressing roller rubs an outer surface of the wall of the flow path 32.

The plurality of pumps 98 includes a first supply pump 102, a first circulation pump 104, a second supply pump 106, and a second circulation pump 108. Note that, as illustrated in Fig. 1, a state in which the cell culture circuit 16 is set in the support device 18 is simply referred to as a "set state".

In the set state, a part of the first supply flow path 56 is attached to the first supply pump 102. The first supply pump 102 applies a flow force in a direction from the supply unit 22 toward the first circulation flow path 58 to the liquid in the first supply flow path 56.

In the set state, a part of the first circulation flow path 58 is attached to the first circulation pump 104. The first circulation pump 104 applies a flow force in a direction from the first outlet port 50 toward the first inlet port 48 to the liquid in the first circulation flow path 58. Note that, the first circulation pump 104 can also apply a flow force in a direction from the first inlet port 48 toward the first outlet port 50 to the liquid in the first circulation flow path 58.

In the set state, a part of the second supply flow path 60 is attached to the second supply pump 106. The second supply pump 106 applies a flow force in a direction from the supply unit 22 toward the second circulation flow path 62 to the liquid in the second supply flow path 60.

In the set state, a part of the second circulation flow path 62 is attached to the second circulation pump 108. The second circulation pump 108 applies a flow force in a direction from the second outlet port 54 toward the second inlet port 52 to the liquid in the second circulation flow path 62. Note that, the second circulation pump 108 can also apply a flow force in a direction from the second inlet port 52 toward the second outlet port 54 to the liquid in the second circulation flow path 62.

The plurality of clamps 100 closes the flow path 32 by pressing the outer surface of the flow path 32 toward an inner surface. For example, the plurality of clamps 100 is open/close valves. The plurality of clamps 100 includes a recovery clamp 110, a first waste liquid clamp 112, a second waste liquid clamp 114, and a third waste liquid clamp 116.

In the set state, a part of the recovery flow path 64 is attached to the recovery clamp 110. The recovery clamp 110 opens and closes the recovery flow path 64. In the set state, a part of the first waste liquid flow path 76 is attached to the first waste liquid clamp 112. The first waste liquid clamp 112 opens and closes the first waste liquid flow path 76. In the set state, a part of the second waste liquid flow path 78 is attached to the second waste liquid clamp 114. The second waste liquid clamp 114 opens and closes the second waste liquid flow path 78. In the set state, a part of the third waste liquid flow path 80 is attached to the third waste liquid clamp 116. The third waste liquid clamp 116 opens and closes the third waste liquid flow path 80.

[1-1-3 Control Unit 20]

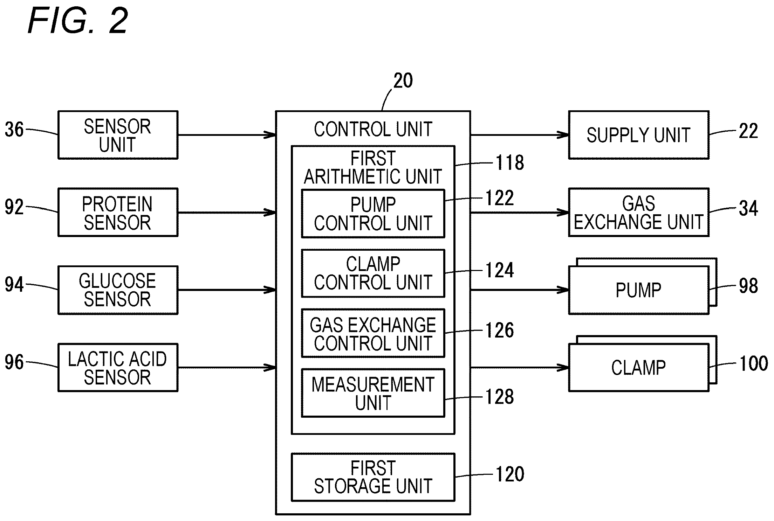

Fig. 2 is a diagram illustrating a configuration of the control unit 20 of the cell culture apparatus 12. The control unit 20 includes a first arithmetic unit 118, a first storage unit 120, and various drive circuits (not illustrated).

The first arithmetic unit 118 includes a processing circuit. The processing circuit may be a processor such as a CPU. The processing circuit may be an integrated circuit such as an ASIC and an FPGA. The processor can execute various types of processing by executing a program stored in the first storage unit 120. The control unit 20 functions as a pump control unit 122, a clamp control unit 124, a gas exchange control unit 126, and a measurement unit 128. At least a part of the plurality of pieces of processing may be executed by an electronic circuit including a discrete device.

The pump control unit 122 controls each of the plurality of pumps 98. Specifically, the pump control unit 122 outputs a command signal to a pump drive circuit (not illustrated). The pump drive circuit supplies power according to the command signal of the pump control unit 122 to each of the plurality of pumps 98. The clamp control unit 124 controls each of the plurality of clamps 100. Specifically, the clamp control unit 124 outputs a command signal to a clamp drive circuit (not illustrated). The clamp drive circuit supplies power according to the command signal of the clamp control unit 124 to each of the plurality of clamps 100. The gas exchange control unit 126 controls the gas exchange unit 34. Specifically, the gas exchange control unit 126 outputs a command signal to a gas exchanger drive circuit (not illustrated). The gas exchanger drive circuit supplies power according to the command signal of the gas exchange control unit 126 to the gas exchange unit 34. The measurement unit 128 acquires the measurement result from each of the gas sensor 88, the pH sensor 90, the protein sensor 92, the glucose sensor 94, and the lactic acid sensor 96. The measurement unit 128 stores the acquired measurement result in the first storage unit 120.

The first storage unit 120 includes a volatile memory and a nonvolatile memory. Examples of the volatile memory include a RAM and the like, for example. The volatile memory is used as a working memory of the processor. The volatile memory temporarily stores data and the like necessary for processing or an arithmetic operation. Examples of the nonvolatile memory include a ROM, a flash memory and the like. The nonvolatile memory is used as a memory for storage. The nonvolatile memory stores a program, a table, a map and the like. At least a part of the first storage unit 120 may be provided in the processor, the integrated circuit and the like described above.

[1-2 Simulation Apparatus 14]

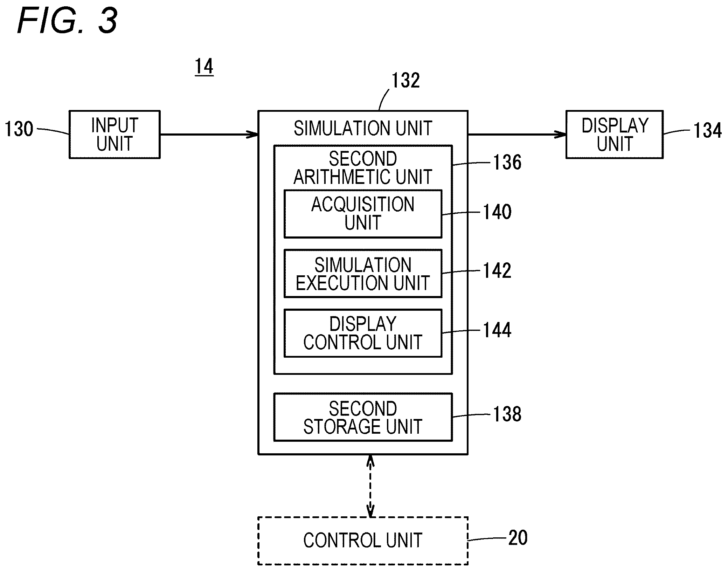

Fig. 3 is a diagram illustrating a configuration of the simulation apparatus 14. The simulation apparatus 14 includes an input unit 130, a simulation unit 132, and a display unit 134. As the simulation apparatus 14, a personal computer, a smartphone, a tablet and the like may be used.

The input unit 130 includes a human-machine interface such as a keyboard, a mouse, and a touch pad. The input unit 130 may include a human-machine interface integrated with the display unit 134 such as a touch panel. The input unit 130 can input data according to an operation performed by the user to the simulation unit 132.

The simulation unit 132 includes a second arithmetic unit 136 and a second storage unit 138. As the second arithmetic unit 136 and the second storage unit 138, the first arithmetic unit 118 and the first storage unit 120 may be used. That is, the control unit 20 of the cell culture apparatus 12 may be used as the simulation unit 132. The second arithmetic unit 136 includes a processing circuit. The processing circuit may be a processor such as a CPU. The processing circuit may be an integrated circuit such as an ASIC and an FPGA. The processor can execute various types of processing by executing a program stored in the second storage unit 138. The simulation unit 132 functions as an acquisition unit 140, a simulation execution unit 142, and a display control unit 144. At least a part of the plurality of pieces of processing may be executed by an electronic circuit including a discrete device.

The acquisition unit 140 acquires data from the outside of the second arithmetic unit 136. For example, the acquisition unit 140 can acquire the data from the input unit 130, the second storage unit 138 and the like. The acquisition unit 140 can acquire the data designated by the input unit 130 from the second storage unit 138. The acquisition unit 140 can acquire the data designated by the input unit 130 from a device (such as the control unit 20) designated by the input unit 130. Using the data acquired by the acquisition unit 140, the simulation execution unit 142 executes a simulation of the cell proliferation and change in concentration of protein by the cell culture apparatus 12. The display control unit 144 allows the display unit 134 to display various screens. For example, the display control unit 144 can allow the display unit 134 to display the data stored in the second storage unit 138. The display control unit 144 can allow the display unit 134 to display information according to a result of the simulation executed by the simulation execution unit 142.

The second storage unit 138 includes a volatile memory and a nonvolatile memory. Examples of the volatile memory include a RAM and the like, for example. The volatile memory is used as a working memory of the processor. The volatile memory temporarily stores data and the like necessary for processing or an arithmetic operation. Examples of the nonvolatile memory include a ROM, a flash memory and the like. The nonvolatile memory is used as a memory for storage. The nonvolatile memory stores a program, a table, a map and the like. In the present embodiment, the nonvolatile memory stores a simulation program executed by the simulation execution unit 142. Furthermore, the nonvolatile memory stores defaults of various data regarding cell proliferation. At least a part of the second storage unit 138 may be provided in the processor, the integrated circuit and the like described above.

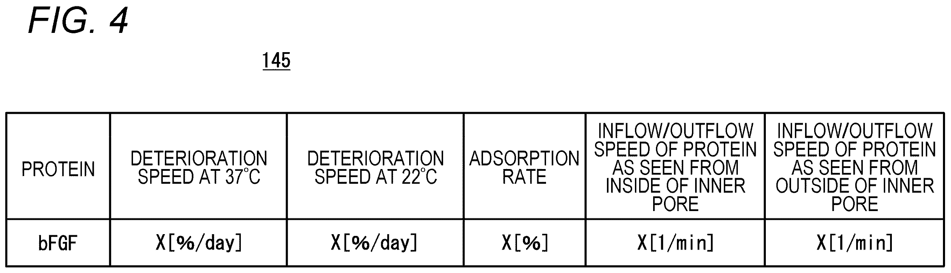

Fig. 4 is a table illustrating parameter information 145. The second storage unit 138 stores the parameter information 145. The parameter information 145 is a data set of parameters used to perform an arithmetic operation regarding a protein in culture simulation. The parameter information 145 includes various types of information ("protein deterioration speed at 37°C (also referred to as deterioration rate, decomposition rate and the like)", "protein deterioration speed at 22°C (also referred to as deterioration rate, decomposition rate and the like)", "protein adsorption rate", "inflow/outflow speed of protein as seen from inside of inner pore", "inflow/outflow speed of protein as seen from outside of inner pore" and the like) corresponding to a protein type. In Fig. 4, the parameter information 145 of bFGF, which is one of growth factors, is illustrated. The second storage unit 138 also stores the parameter information 145 corresponding to proteins other than bFGF.

Note that, the "protein deterioration speed at 37°C" is a protein deterioration speed caused by temperature around the first circulation flow path 58. This is a parameter on the premise that the first circulation flow path 58 is placed in a temperature environment at 37°C. In a case where the first circulation flow path 58 is placed in another temperature environment, a parameter corresponding to the temperature environment is set.

Similarly, the "protein deterioration speed at 22°C" is a protein deterioration speed caused by temperature around the first supply unit 22a. This is a parameter on the premise that the first supply unit 22a is placed in a temperature environment at 22°C. In a case where the first supply unit 22a is placed in another temperature environment, a parameter corresponding to the temperature environment is set.

The display unit 134 includes a human-machine interface such as a display. The display unit 134 may include a human-machine interface integrated with the input unit 130 like a touch panel. The display unit 134 can display various screens described in the following [2].

[2 Screen]

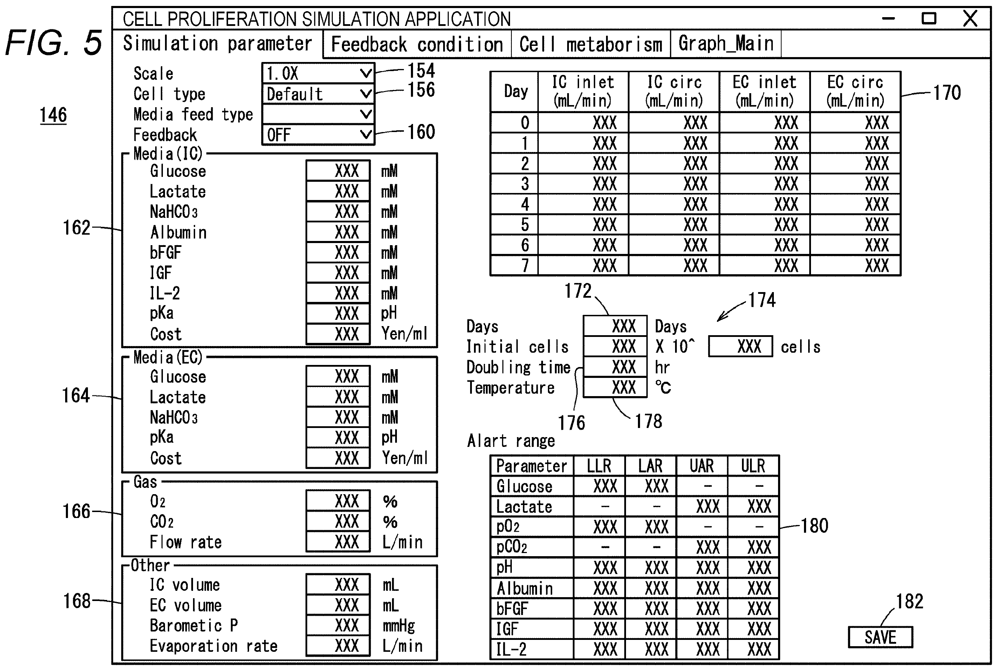

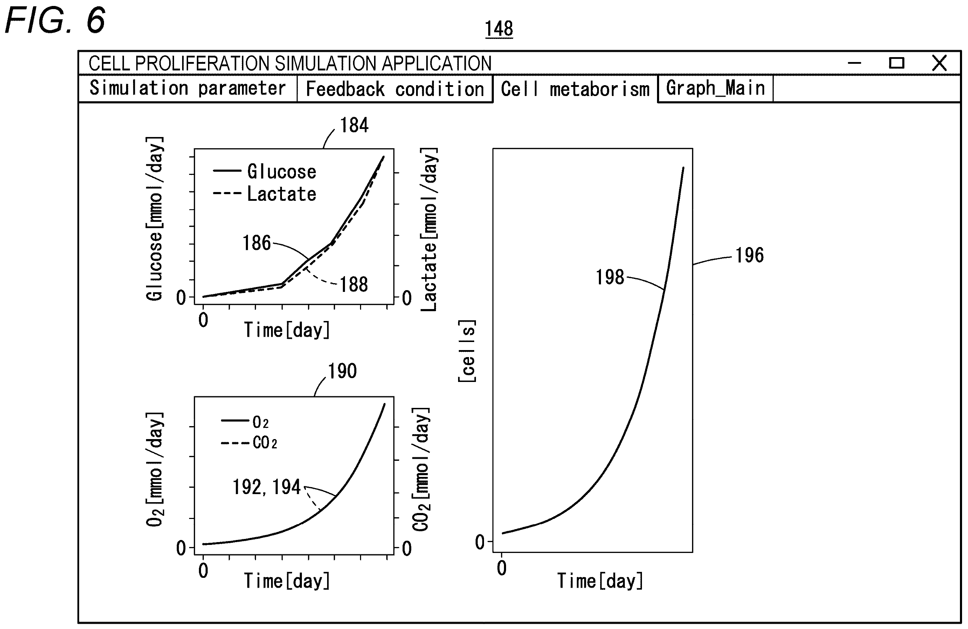

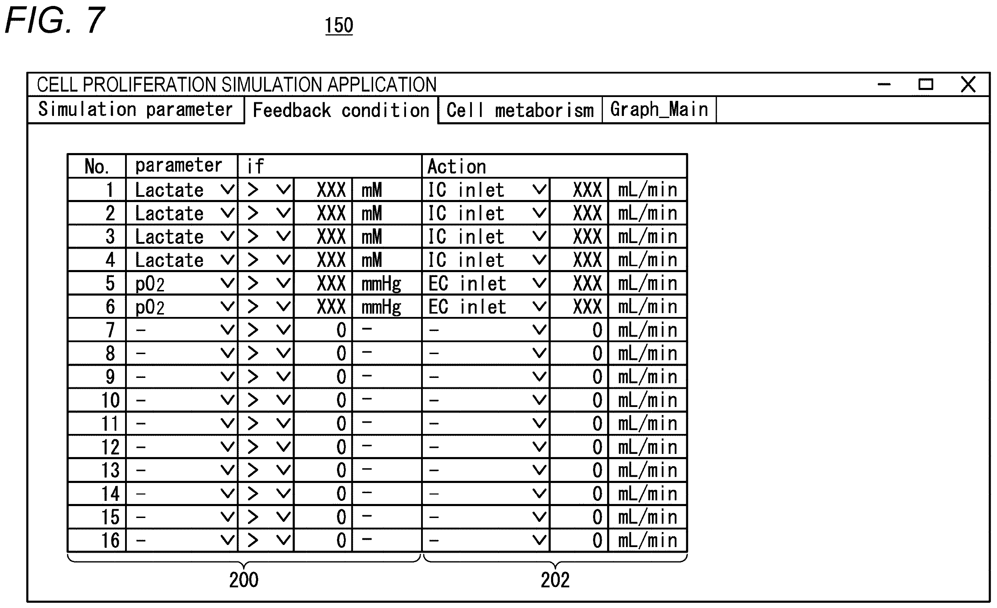

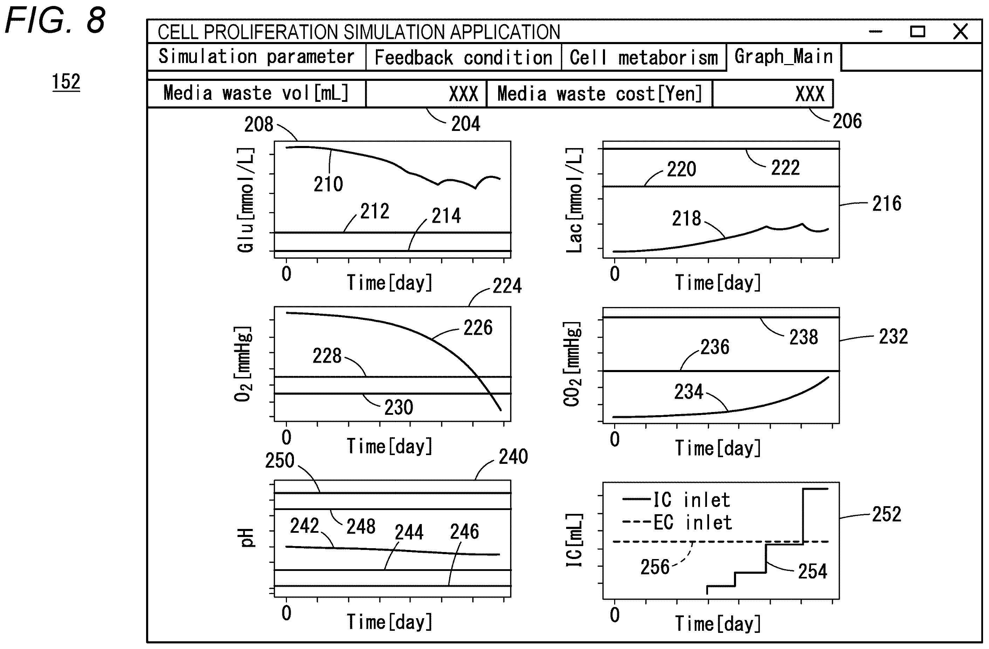

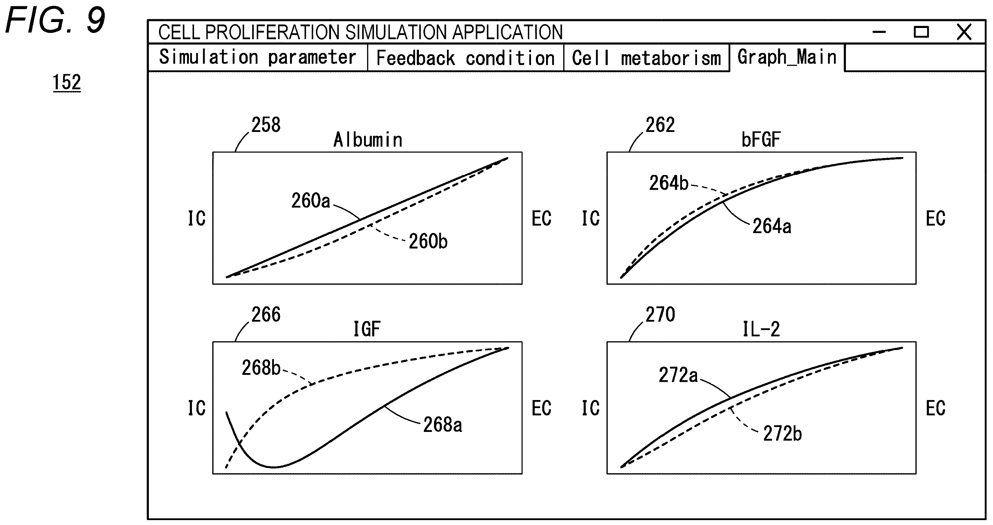

The display unit 134 can display an input screen 146 (Fig. 5), a proliferation data screen 148 (Fig. 6), a feedback condition screen 150 (Fig. 7), a result screen 152 (Figs. 8 and 9) and the like.

[2-1 Input screen 146]

Fig. 5 is a diagram illustrating the input screen 146 displayed on the display unit 134. The input screen 146 is a screen for inputting various data used in cell culture simulation. When the user operates the input unit 130, the display unit 134 displays the input screen 146.

The input screen 146 includes a scale field 154. The scale field 154 is an input field for designating a scale of cell culture in simulation. The user can select a scale from a drop-down list displayed in the scale field 154.

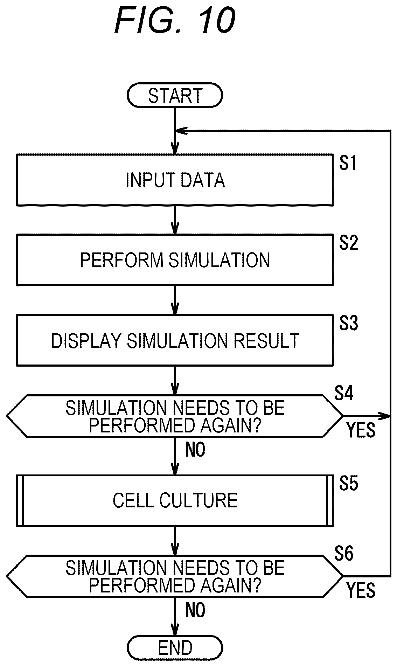

The input screen 146 includes a cell type field 156. The cell type field 156 is an input field for designating proliferation data used in the simulation. The proliferation data is data indicating a cell proliferation state under any culture condition. The proliferation data designated in the cell type field 156 is a proliferation model of cells for simulation. The proliferation data is created on the basis of data actually measured at a cell culture step performed in the past. The second storage unit 138 stores a default of the proliferation data. The second storage unit 138 can store data actually measured at the cell culture step illustrated at step S5 in Fig. 10 as the proliferation data. A specific example of the proliferation data is illustrated in Fig. 6. The user can select either the default or an actual measurement result from a drop-down list displayed in the cell type field 156.

The input screen 146 includes a feedback field 160. The feedback field 160 is an input field for designating whether to use a feedback condition in the simulation. A specific example of the feedback condition is illustrated in Fig. 7. The user can select either "ON" or "OFF" from a drop-down list displayed in the feedback field 160. When "ON" is selected, the feedback condition is used in the simulation. When "OFF" is selected, the feedback condition is not used in the simulation.

The input screen 146 includes a complete medium input field 162. The complete medium input field 162 is an input field for designating data of the complete medium to be circulated in the first circulation flow path 58 in the simulation. Examples of the data of the complete medium include the glucose concentration, the lactic acid concentration, the concentration of one or more proteins, the type of the complete medium, pka, a unit price of the complete medium and the like. The protein may be of one type or a plurality of types. The user can designate the protein to be added to the complete medium in the complete medium input field 162. The data of the complete medium is condition data indicating a culture condition of the simulation.

The input screen 146 includes a basal medium input field 164. The basal medium input field 164 is an input field for designating data of the basal medium to be circulated in the second circulation flow path 62 in the simulation. Examples of the data of the basal medium include the glucose concentration, the lactic acid concentration, the type of the basal medium, pka, a unit price of the basal medium and the like. The data of the basal medium is condition data indicating a culture condition of the simulation.

The input screen 146 includes a gas input field 166. The gas input field 166 is an input field for designating data of the gas to be used in the gas exchange unit 34 in the simulation. Examples of the data of the gas include a volume ratio of oxygen contained in the gas, a volume ratio of carbon dioxide contained in the gas, a flow rate of the gas and the like. The data of the gas is condition data indicating a culture condition of the simulation.

The input screen 146 includes other input field 168. The other input field 168 is an input field for designating other data regarding the culture medium. The other data includes a capacity of the first circulation flow path 58, a capacity of the second circulation flow path 62, an atmospheric pressure, a water vapor pressure and the like. The other data is condition data indicating a culture condition of the simulation.

The input screen 146 includes a pump speed input field 170. The pump speed input field 170 is an input field for designating a flow rate of each pump 98 in the simulation. The flow rate of each pump 98 is set for each day of the culture period. The flow rate of each pump 98 is condition data indicating a culture condition of the simulation.

The input screen 146 includes a day count input field 172. The day count input field 172 is an input field for designating the number of culture days of the cells in the simulation. The number of culture days is condition data indicating a culture condition of the simulation.

The input screen 146 includes a seeding times input field 174. The seeding times input field 174 is an input field for designating the number of times of seeding in the simulation. The number of times of seeding is condition data indicating a culture condition of the simulation.

The input screen 146 includes a doubling time input field 176. The doubling time input field 176 is an input field for designating a time (doubling time) in which the cell doubles in the simulation. The doubling time is condition data indicating a culture condition of the simulation.

The input screen 146 includes a temperature input field 178. The temperature input field 178 is an input field for designating environmental temperature in the simulation. The environmental temperature is condition data indicating a culture condition of the simulation.

The input screen 146 includes a threshold input field 180. The threshold input field 180 is an input field for designating a threshold of each of the glucose concentration, the lactic acid concentration, a partial pressure of oxygen, a partial pressure of carbon dioxide, the pH, and protein. As the threshold, at least one of a lower limit value (LLR), a lower alert value (LAR), an upper limit value (ULR), and an upper alert value (UAR) is designated. For example, it is possible that only the lower limit value (LLR) and the lower alert value (LAR) are designated. For example, it is possible that only the upper limit value (ULR) and the upper alert value (UAR) are designated. For example, it is possible that the lower limit value (LLR), the lower alert value (LAR), the upper limit value (ULR), and the upper alert value (UAR) are designated. The user can optionally designate the threshold.

[2-2 Proliferation Data Screen 148]

Fig. 6 is a diagram illustrating the proliferation data screen 148 displayed on the display unit 134. The proliferation data screen 148 is a screen illustrating each piece of proliferation data. When the user operates the input unit 130, the display unit 134 displays the proliferation data screen 148. The second storage unit 138 stores each piece of proliferation data as a data set.

The proliferation data screen 148 includes a biodata graph 184. In the biodata graph 184, the horizontal axis represents time, and the vertical axis represents a metabolic speed of biodata. In the biodata graph 184, a metabolic speed line 186 and a metabolic speed line 188 are displayed. The metabolic speed line 186 indicates transition of the metabolic speed of glucose. The metabolic speed line 188 indicates transition of the metabolic speed of lactic acid. The metabolic speed of the biodata is the proliferation data indicating the cell proliferation state.

The proliferation data screen 148 includes a gas data graph 190. In the gas data graph 190, the horizontal axis represents time, and the vertical axis represents the metabolic speed of the biodata. In the gas data graph 190, a metabolic speed line 192 and a metabolic speed line 194 are displayed. The metabolic speed line 192 indicates transition of the metabolic speed of oxygen. The metabolic speed line 194 indicates transition of the metabolic speed of carbon dioxide. The metabolic speed of the gas data is the proliferation data indicating the cell proliferation state.

The proliferation data screen 148 includes a cell graph 196. In the cell graph 196, the horizontal axis represents time and the vertical axis represents the number of cells. In the cell graph 196, a cell count line 198 is displayed. The cell count line 198 indicates transition of the number of cells. The number of cells is the proliferation data indicating the cell proliferation state.

[2-3 Feedback Condition Screen 150]

Fig. 7 is a diagram illustrating a feedback condition screen 150 displayed on the display unit 134. The feedback condition screen 150 is a screen for inputting the feedback condition and change data. The feedback condition is a condition for changing the condition data according to the situation of the simulation during the simulation. The change data is a change value of the condition data. When the user operates the input unit 130, the display unit 134 displays the feedback condition screen 150.

The feedback condition screen 150 includes a condition field 200 and a data field 202. The condition field 200 is an input field for designating the feedback condition. The data field 202 is an input field for designating the change data. For example, the condition field 200 and the data field 202 indicated by No. 1 in Fig. 7 mean that "the flow rate of the first circulation pump 104 is set to XXX [mL/min] in a case where lactic acid is more than XXX [mM]". The feedback condition and the change data are the condition data indicating the culture condition of the simulation. Note that, although not illustrated, the feedback condition of glucose, carbon dioxide, pH, various proteins and the like can also be designated.

When a save button 182 on the input screen 146 illustrated in Fig. 5 is pressed, the second storage unit 138 stores data designated in each input field on the feedback condition screen 150.

[2-4 Result screen 152]

Figs. 8 and 9 are diagrams illustrating the result screen 152 displayed on the display unit 134. Fig. 8 illustrates an upper portion of the result screen 152, and Fig. 9 illustrates a lower portion of the result screen 152. When the user performs a downward scroll operation while the result screen 152 in Fig. 8 is displayed on the display unit 134, the result screen 152 in Fig. 9 is displayed on the display unit 134. The result screen 152 is a screen illustrating a result of the simulation performed at step S2 in Fig. 10. After the simulation, when the user operates the input unit 130, the display control unit 144 allows the display unit 134 to display the result screen 152.

The result screen 152 includes a discarded amount field 204 and a cost field 206. The total discarded amount of the culture medium in the simulated cell culture is displayed in the discarded amount field 204. A cost in the simulated cell culture is displayed in the cost field 206.

The result screen 152 includes a glucose graph 208 (Fig. 8). In the glucose graph 208, the horizontal axis represents time, and the vertical axis represents the glucose concentration. In the glucose graph 208, a concentration line 210, an alert line 212, and a lower limit line 214 are displayed. The concentration line 210 indicates transition of the glucose concentration during the culture period. The alert line 212 indicates a boundary value between an OK range and an alert range. The lower limit line 214 indicates a boundary value between the alert range and an NG range. The boundary value indicated by the alert line 212 is a lower alert value of the glucose concentration input in the threshold input field 180 of the input screen 146. The boundary value indicated by the lower limit line 214 is a lower limit value of the glucose concentration input in the threshold input field 180 of the input screen 146. A range above the alert line 212 is the OK range. A range below the lower limit line 214 is the NG range. A range between the alert line 212 and the lower limit line 214 is the alert range. The concentration line 210 is preferably in the OK range above the alert line 212. That is, the glucose concentration is preferably within the OK range at all times during the culture period.

The result screen 152 includes a lactic acid graph 216 (Fig. 8). In the lactic acid graph 216, the horizontal axis represents time, and the vertical axis represents the lactic acid concentration. In the lactic acid graph 216, a concentration line 218, an alert line 220, and an upper limit line 222 are displayed. The concentration line 218 indicates transition of the lactic acid concentration during the culture period. The alert line 220 indicates a boundary value between an OK range and an alert range. The upper limit line 222 indicates a boundary value between the alert range and an NG range. The boundary value indicated by the alert line 220 is an upper alert value of the lactic acid concentration input in the threshold input field 180 of the input screen 146. The boundary value indicated by the upper limit line 222 is an upper limit value of the lactic acid concentration input in the threshold input field 180 of the input screen 146. A range below the alert line 220 is an OK range. A range above the upper limit line 222 is an NG range. A range between the alert line 220 and the upper limit line 222 is an alert range. The concentration line 218 is preferably in the OK range below the alert line 220. That is, the lactic acid concentration is preferably within the OK range at all times during the culture period.

The result screen 152 includes an $O_2$ graph 224 (Fig. 8). In the $O_2$ graph 224, the horizontal axis represents time, and the vertical axis represents a partial pressure of oxygen. In the $O_2$ graph 224, a partial pressure line 226, an alert line 228, and a lower limit line 230 are displayed. The partial pressure line 226 indicates transition of the partial pressure of oxygen during the culture period. The alert line 228 indicates a boundary value between an OK range and an alert range. The lower limit line 230 indicates a boundary value between the alert range and an NG range. The boundary value indicated by the alert line 228 is a lower alert value of the partial pressure of oxygen input in the threshold input field 180 of the input screen 146. The boundary value indicated by the lower limit line 230 is a lower limit value of the partial pressure of oxygen input in the threshold input field 180 of the input screen 146. A range above the alert line 228 is an OK range. A range below the lower limit line 230 is an NG range. A range between the alert line 228 and the lower limit line 230 is an alert range. The partial pressure line 226 is preferably in the OK range above the alert line 228. That is, the partial pressure of oxygen is preferably within the OK range at all times during the culture period.

The result screen 152 includes a $CO_2$ graph 232 (Fig. 8). In the $CO_2$ graph 232, the horizontal axis represents time, and the vertical axis represents the partial pressure of carbon dioxide. In the $CO_2$ graph 232, a partial pressure line 234, an alert line 236, and an upper limit line 238 are displayed. The partial pressure line 234 indicates transition of the partial pressure of carbon dioxide during the culture period. The alert line 236 indicates a boundary value between an OK range and an alert range. The upper limit line 238 indicates a boundary value between the alert range and an NG range. The boundary value indicated by the alert line 236 is an upper alert value of partial pressure of carbon dioxide input in the threshold input field 180 of the input screen 146. The boundary value indicated by the upper limit line 238 is an upper limit value of the partial pressure of carbon dioxide input in the threshold input field 180 of the input screen 146. A range below the alert line 236 is an OK range. A range above the upper limit line 238 is an NG range. A range between the alert line 236 and the upper limit line 238 is an alert range. The partial pressure line 234 is preferably in the OK range below the alert line 236. That is, the partial pressure of carbon dioxide is preferably within the OK range at all times during the culture period.

The result screen 152 includes a pH graph 240 (Fig. 8). In the pH graph 240, the horizontal axis represents time, and the vertical axis represents the pH of the culture medium. In the pH graph 240, a pH line 242, a lower alert line 244, a lower limit line 246, an upper alert line 248, and an upper limit line 250 are displayed. The pH line 242 indicates transition of the pH during the culture period. The lower alert line 244 indicates a boundary value between an OK range and a lower alert range. The lower limit line 246 indicates a boundary value between the lower alert range and a first NG range. The upper alert line 248 indicates a boundary value between the OK range and an upper alert range. The upper limit line 250 indicates a boundary value between the upper alert range and a second NG range. The boundary value indicated by the lower alert line 244 is a lower alert value of the pH input in the threshold input field 180 of the input screen 146. The boundary value indicated by the lower limit line 246 is a lower limit value of the pH input in the threshold input field 180 of the input screen 146. The boundary value indicated by the upper alert line 248 is an upper alert value of the pH input in the threshold input field 180 of the input screen 146. The boundary value indicated by the upper limit line 250 is an upper limit value of the pH input in the threshold input field 180 of the input screen 146. A range between the lower alert line 244 and the upper alert line 248 is an OK range. A range below the lower limit line 246 is the first NG range. A range between the lower alert line 244 and the lower limit line 246 is a lower alert range. The range above the upper limit line 250 is the second NG range. A range between the upper alert line 248 and the upper limit line 250 is the upper alert range. The pH line 242 is preferably in the OK range between the lower alert line 244 and the upper alert line 248. That is, the pH of the culture medium is preferably within the OK range at all times during the culture period.

The result screen 152 includes a flow rate graph 252 (Fig. 8). In the flow rate graph 252, the horizontal axis represents time, and the vertical axis represents the flow rate of the first circulation pump 104 and the flow rate of the second circulation pump 108. In the flow rate graph 252, a first flow rate line 254 and a second flow rate line 256 are displayed. The first flow rate line 254 indicates transition of the flow rate of the first circulation pump 104 during the culture period. The second flow rate line 256 indicates transition of the flow rate of the second circulation pump 108 during the culture period.

The result screen 152 includes an albumin graph 258 (Fig. 9). In the albumin graph 258, the horizontal axis represents time and the vertical axis represents concentration. Note that, in the drawing, the left vertical axis (IC) represents the concentration in the first circulation flow path 58, and the right vertical axis (EC) represents an albumin concentration in the second circulation flow path 62. This is similar in other graphs in Fig. 9. Concentration lines 260a and 260b are displayed in the albumin graph 258. The concentration line 260a indicates transition of the albumin concentration in the first circulation flow path 58 during the culture period. The concentration line 260b indicates transition of the albumin concentration in the second circulation flow path 62 during the culture period. Note that, although not illustrated, a lower alert line, a lower limit line, an upper alert line, an upper limit line and the like may also be illustrated in the albumin graph 258 similarly to each graph in Fig. 8. This is similar in other graphs in Fig. 9.

The result screen 152 includes a bFGF graph 262 (Fig. 9). In the bFGF graph 262, concentration lines 264a and 264b are displayed. The concentration line 264a indicates transition of a bFGF concentration in the first circulation flow path 58 during the culture period. The concentration line 264b indicates transition of the bFGF concentration in the second circulation flow path 62 during the culture period.

The result screen 152 includes an IGF graph 266 (Fig. 9). In the IGF graph 266, concentration lines 268a and 268b are displayed. The concentration line 268a indicates transition of an IGF concentration in the first circulation flow path 58 during the culture period. The concentration line 268b indicates transition of the IGF concentration in the second circulation flow path 62 during the culture period.

The result screen 152 includes an IL-2 graph 270 (Fig. 9). In the IL-2 graph 270, concentration lines 272a and 272b are displayed. The concentration line 272a indicates transition of an IL-2 concentration in the first circulation flow path 58 during the culture period. The concentration line 272b indicates transition of the IL-2 concentration in the second circulation flow path 62 during the culture period.

Note that, the result screen 152 can also illustrate transition of concentrations of other proteins. In short, the result screen 152 can illustrate transition of the concentration of the protein designated in the complete medium input field 162 of the input screen 146.

[3 Cell Culture Method]

[3-1 Entire Flow of Cell Culture Method]

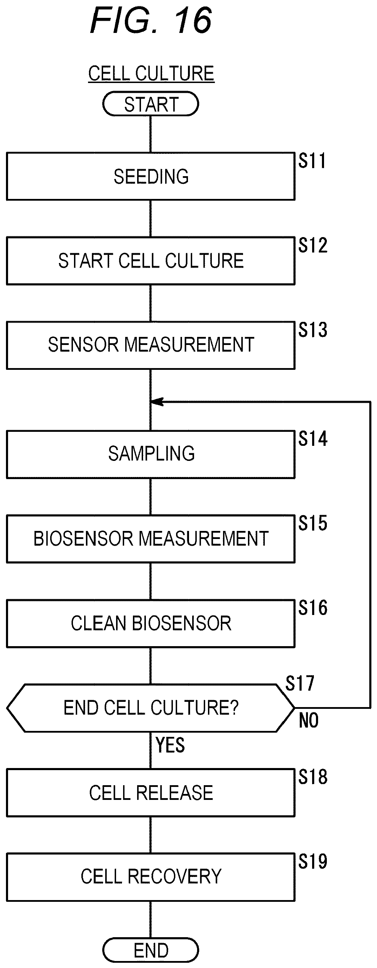

Fig. 10 is a flowchart illustrating a flow of a cell culture method performed using the cell culture system 10. Steps S1 to S3 in Fig. 10 are performed by the simulation apparatus 14. Step S5 in Fig. 10 is performed by the cell culture apparatus 12. Step S5 is illustrated in detail in Fig. 16. Steps S4 and S6 are determined by the user.

Before step S1, the user operates the input unit 130 to start the simulation program. The second arithmetic unit 136 executes the simulation program stored in the second storage unit 138 in response to the operation of the user. Then, the display control unit 144 allows the display unit 134 to display the input screen 146 illustrated in Fig. 5.

At step S1, the user operates the input unit 130 to input data in each input field of the input screen 146. For example, in initial simulation, the user designates default in the cell type field 156. The user presses the save button 182 after designating each piece of data. The input unit 130 inputs data in each input field to the simulation unit 132. The second storage unit 138 stores each piece of data. When step S1 ends, the processing proceeds to step S2.

At step S2, the user operates the input unit 130 to start cell culture simulation. In response to the instruction from the input unit 130, the simulation execution unit 142 starts the cell culture simulation using each piece of data (proliferation data, condition data, and various parameters of protein) stored in the second storage unit 138. The simulation execution unit 142 simulates the cell culture during a designated culture period using the proliferation data, the condition data, and various parameters of protein. The simulation execution unit 142 calculates the amount of each component in the culture medium at each time during the culture period. Specifically, the simulation execution unit 142 calculates the glucose concentration at each time during the culture period. The simulation execution unit 142 calculates the lactic acid concentration at each time during the culture period. The simulation execution unit 142 calculates the partial pressure of oxygen at each time during the culture period. The simulation execution unit 142 calculates the partial pressure of carbon dioxide at each time during the culture period. The simulation execution unit 142 calculates the pH of the culture medium at each time during the culture period. The simulation execution unit 142 can calculate the amount of each component by a known arithmetic method. The arithmetic method is disclosed in, for example, the literature "Journal of Chemical Technology and Metallurgy, 48, 4, 2013, 351-356 EXPERIMENTAL DETERMINATION OF THE VOLUMETRIC MASS TRANSFER COEFFICIENT". The simulation execution unit 142 calculates the concentration of protein at each time during the culture period. Note that, a method of calculating the concentration of protein performed by the simulation execution unit 142 will be described in [3-2] below. The second storage unit 138 stores an arithmetic result of the simulation execution unit 142.

The simulation execution unit 142 determines whether the feedback condition is satisfied on the basis of each arithmetic value at each time. In a case where the feedback condition is satisfied, the simulation execution unit 142 changes a part of the culture condition according to setting of the feedback condition. For example, the simulation execution unit 142 changes the data of the flow rate of any one of the pumps 98. The simulation execution unit 142 continues the simulation using the changed data. The second storage unit 138 stores the changed condition data.

When the simulation of the cell culture ends, the simulation execution unit 142 calculates the total consumption amount and the total discarded amount of the culture medium in the simulation. The simulation execution unit 142 calculates a cost by using the total consumption amount of the culture medium and the unit price of the culture medium. The second storage unit 138 stores an arithmetic result of the simulation execution unit 142. When step S2 ends, the processing proceeds to step S3.

At step S3, the user operates the input unit 130 to display the result of the simulation. In response to the instruction from the input unit 130, the display control unit 144 allows the display unit 134 to display the result of the simulation. The display unit 134 displays the result screen 152 illustrated in Figs. 8 and 9. When step S3 ends, the processing proceeds to step S4.

At step S4, the user determines whether the simulation needs to be performed again. In any graph of the result screen 152, in a case where there is a part deviating from the OK range in the transition of the arithmetic value, the user preferably changes the condition data and executes the simulation again. In a case where the simulation needs to be performed again (step S4: YES), the processing returns to step S1. In contrast, in a case where the simulation does not need to be performed again (step S4: NO), the processing proceeds to step S5.

At step S5, the user performs the cell culture using the cell culture apparatus 12. The user operates an input device (not illustrated) of the cell culture apparatus 12 to set the culture condition designated at step S1 in Fig. 10. For example, in a case where the control unit 20 of the cell culture apparatus 12 and the simulation unit 132 of the simulation apparatus 14 are connected by a signal line, the control unit 20 acquires the condition data of the culture condition from the second storage unit 138 of the simulation unit 132. Processing of the cell culture will be described in [3-3] below. Note that, after the processing of the cell culture ends, the simulation unit 132 acquires the condition data of the culture condition and the proliferation data of a new cell from the control unit 20. The second storage unit 138 stores each piece of data acquired from the control unit 20. When step S5 ends, the processing proceeds to step S6.

At step S6, the user determines whether the simulation needs to be performed again. The cell culture is performed a plurality of times. The user increases the scale of the cell culture stepwise as the number of times of cell culture increases. The user preferably performs the simulation each time the scale of the cell culture is increased. In a case where the simulation needs to be performed again (step S6: YES), the processing returns to step S1. In contrast, in a case where the simulation does not need to be performed again (step S6: NO), the cell culture ends.

Note that, at step S6, the user may compare the result of the simulation performed at step S3 with the measurement result in the cell culture performed at step S5. In this case, the display control unit 144 may allow the display unit 134 to display a predicted value of the protein and the like obtained by the simulation and an actually measured value of the protein and the like obtained by actual cell culture. Furthermore, the display control unit 144 may allow the display unit 134 to display a range of $\pm5\%\times$day with respect to the actually measured value as an allowable range of the protein and the like. In this case, the display control unit 144 calculates a value of -5%×day with respect to the actually measured value, and sets the same as a lower limit value of the allowable range. The display control unit 144 calculates a value of +5%×day with respect to the actually measured value, and sets the same as an upper limit value of the allowable range.

[3-2 Method of Calculating Protein Concentration]

The simulation execution unit 142 calculates the transition of the concentration of protein during the designated culture period. The simulation execution unit 142 calculates the concentration every predetermined time as the transition of the concentration of protein. For example, the simulation execution unit 142 calculates the concentration at one-minute intervals. Note that, an interval at which the concentration of protein is calculated is not limited thereto. For example, the interval at which the concentration of protein is calculated may be a one-second interval or a one-day interval.

The simulation execution unit 142 calculates the transition of the concentration of protein by the calculation method according to the culture form in which the simulation is executed. For example, the simulation execution unit 142 can execute simulations in eight culture forms (first to eighth culture forms) as follows. The first culture form, the third culture form, the fifth culture form, and the seventh culture form are executed in a culture process of floating cells. The second culture form, the fourth culture form, the sixth culture form, and the eighth culture form are executed in a culture process of adherent cells. In each culture process of the floating cells and the adherent cells, one culture form among the plurality of culture forms described above may be executed, or a plurality of culture forms may be combined.

For example, the first culture form and the second culture form are mainly executed from an initial stage to a middle stage of the cell culture process. The third culture form and the fourth culture form are mainly executed from the middle stage to a later stage of the cell culture process. The fifth culture form is appropriately executed during the culture period in order to collect cells. Each culture form is different in a supplied culture medium, a method of supplying the culture medium, a method of discarding the culture medium and the like. The simulation execution unit 142 can execute the simulation obtained by combining a plurality of culture forms in a case of executing the simulation of culture in any period.

The simulation execution unit 142 can use each of a calculation expression of the protein concentration in the first circulation flow path 58 (refer to below) and a calculation expression of the protein concentration in the second circulation flow path 62 (refer to below) determined in each culture form and connect the data obtained by calculating the protein concentration in each culture period by each culture form, thereby obtaining the simulation result of the entire culture period. For example, the simulation execution unit 142 can calculate data indicating the concentration for each culture period by using the calculation expression of the protein concentration of the first culture form, the third culture form, and the fifth culture form performed in the culture of the floating cells and connect the calculated data, thereby obtaining the simulation result of the entire culture period. For example, the simulation execution unit 142 can calculate data indicating the concentration for each culture period by using the calculation expression of the protein concentration of the second culture form and the fourth culture form performed in the culture of the adherent cells and connect the calculated data, thereby obtaining the simulation result of the entire culture period.

[3-2-1 Calculation Method in First Culture Form]

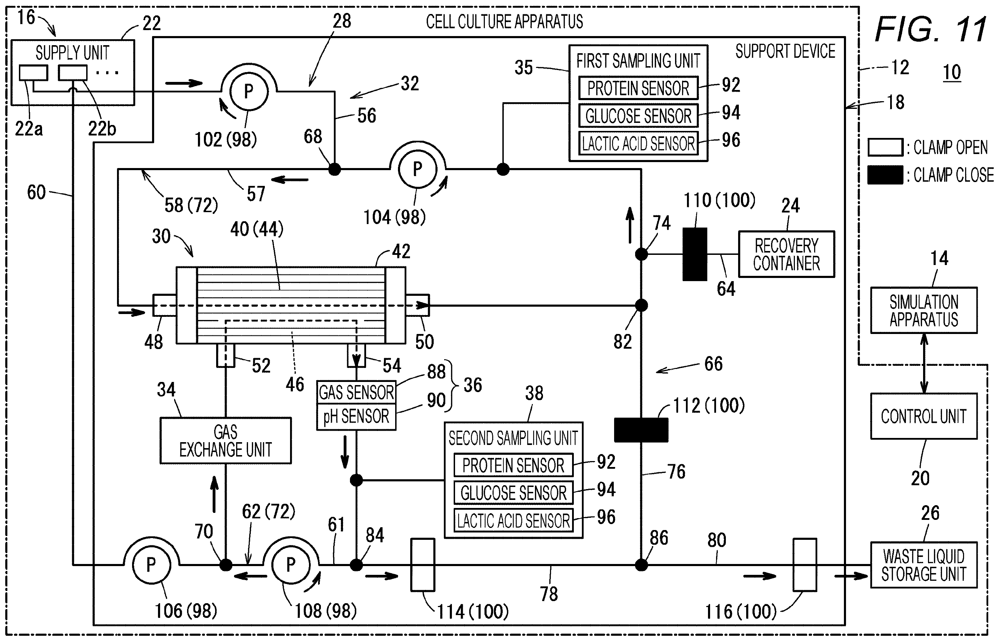

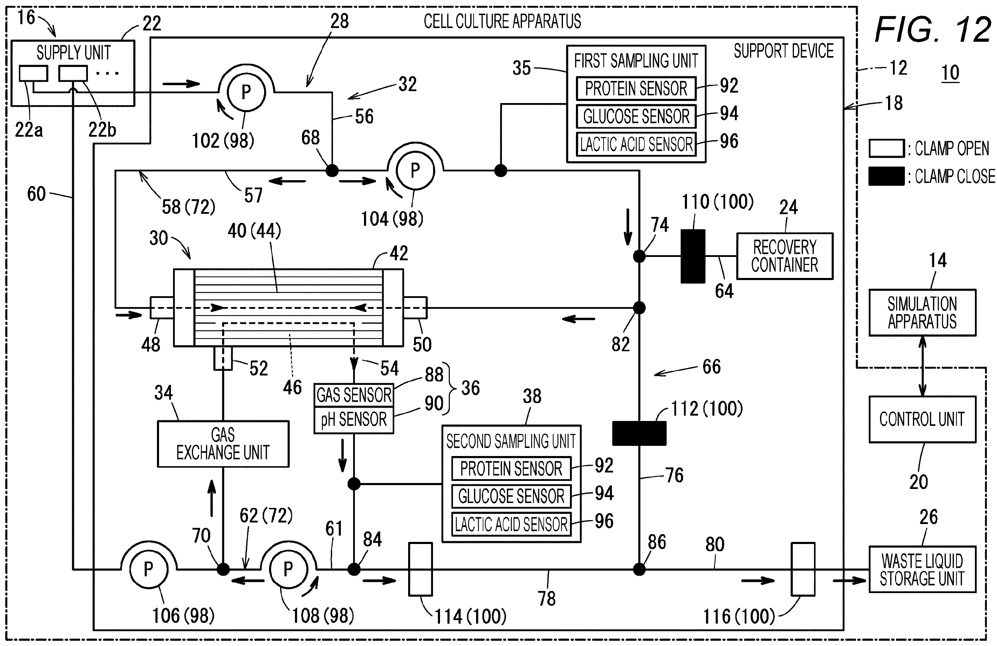

Fig. 11 is a diagram illustrating an operation of the cell culture apparatus 12 at the time of cell culture in the first culture form. Fig. 12 is a diagram illustrating an operation of the cell culture apparatus 12 at the time of cell collection in the first culture form. In the first culture form, the control unit 20 controls the first supply unit 22a, the second supply unit 22b, each pump 98, and each clamp 100 to adjust the flow path through which the culture medium flows and the flow rate of the culture medium. Similarly for the second to eighth culture forms to be described later, the control unit 20 adjusts the flow path through which the culture medium flows and the flow rate of the culture medium. The first culture form is as follows.

(a) The complete medium is supplied from the first supply unit 22a to the first circulation flow path 58.
(b) The basal medium is not supplied from the second supply unit 22b to the second circulation flow path 62.
(c) A part of the culture medium is discarded from the second circulation flow path 62 to the waste liquid storage unit 26 via the second waste liquid flow path 78.

The simulation execution unit 142 calculates the concentration of protein (Pro_IC [n+1]) in the first circulation flow path 58 using the following expressions (1) to (5). The simulation execution unit 142 calculates the concentration of protein (Pro_EC [n+1]) in the second circulation flow path 62 using the following expressions (6) to (9).

[Math. 1]

$$\frac{Pro\_IC[n+1] - Pro\_IC[n]}{t[n+1] - t[n]} = F\_i\_b + L\_b\_c + E\_t\_m1 + L\_i\_b1 \quad (1)$$

$$F\_i\_b = \frac{Cm \times (1 - A\_r) \times (1 - Deg\_22 \times t\_[n]) \times IC\_ir}{V\_IC} \quad (2)$$

$$L\_b\_c = \frac{Mx\_pro[n]}{V\_IC} \quad (3)$$

$$E\_t\_m1 = k\_EtoI \times (Pro\_EC[n] - Pro\_IC[n]) \quad (4)$$

$$L\_i\_b1 = Pro\_IC[n] \times (-Deg\_37) \quad (5)$$

[Math. 2]

$$\frac{Pro\_EC[n+1] - Pro\_EC[n]}{t[n+1] - t[n]} = L\_t\_w2 + E\_t\_m2 + L\_i\_b2 \quad (6)$$

$$L\_t\_w2 = -\frac{Pro\_EC[n] \times IC\_ir}{V\_EC} \quad (7)$$

$$E\_t\_m2 = k\_ItoE \times (Pro\_IC[n] - Pro\_EC[n]) \quad (8)$$

$$L\_i\_b2 = Pro\_EC[n] \times (-Deg\_37) \quad (9)$$

The variables included in expressions (1) to (9) above are defined as follows. Note that, the variable denoted by [n] is a variable obtained in an n-th calculation among calculations performed at one-minute intervals. n is a numerical value from zero to (length of culture period/interval of calculation time).

Pro_IC: concentration of protein in first circulation flow path 58 [unit: ng/mL].

Pro_EC: concentration of protein in second circulation flow path 62 [unit: ng/mL].

t: elapsed time in simulation [unit: min].

Cm: concentration of protein in complete medium supplied from first supply unit 22a to first circulation flow path 58 [unit: ng/mL].

A_r: rate at which protein disappears from culture medium of first supply flow path 56 and first circulation flow path 58 due to protein adsorption, lamination and the like on inner surface of hollow fiber membrane 40 [unit: %].

Deg_22: protein deterioration rate (disappearance rate) caused by ambient temperature (22°C) in first supply unit 22a [%/day].

Deg_37: protein deterioration rate (disappearance rate) caused by ambient temperature (37°C) in first circulation flow path 58 [%/day].

IC_ir: flow rate of first supply pump 102 [unit: mL/min].

V_IC: volume of first circulation flow path 58 [unit: mL].

V_EC: volume of second circulation flow path 62 [unit: mL].

Mx_pro: consumption speed per unit time of protein consumed by cells [unit: mg/min].

k_EtoI: speed (coefficient) at which protein in culture medium flows in and out between inside and outside of inner pore via hollow fiber membrane 40 as seen from inside of inner pore [unit: 1/min].

k_ItoE: speed (coefficient) at which protein in culture medium flows in and out between inside and outside of inner pore via hollow fiber membrane 40 as seen from outside of inner pore [unit: 1/min].

Note that, A_r is also referred to as an adsorption rate or a correction rate. In other words, the adsorption rate (correction rate) is a rate of protein that cannot be consumed by cells because protein is adsorbed, aggregated, and deposited on at least one of the inside of the first circulation flow path (inner circulation path) 58 and the inside of the second circulation flow path (outer circulation path) 62.

Each item included in expression (1) above is defined as follows.

Pro_IC[n+1]-Pro_IC[n]/t[n+1]-t[n]: change speed of concentration of protein in first circulation flow path 58 between timing of n-th calculation and timing of (n+1)-th calculation.

F_i_b: speed (supply speed) at which concentration of protein changes in first circulation flow path 58 due to supply of culture medium to first circulation flow path 58.

L_b_c: speed (consumption speed) at which concentration of protein changes in first circulation flow path 58 due to consumption of protein by cells.

E_t_m1: speed (exchange speed) at which concentration of protein changes in first circulation flow path 58 due to inflow and outflow of culture medium between inside and outside of inner pore via hollow fiber membrane 40. For this concentration, the volume of the first circulation flow path 58 is used as a denominator.

L_i_b1: speed (deterioration speed) at which concentration of protein changes in first circulation flow path 58 due to protein deterioration in first circulation flow path 58.

Each item included in expression (6) above is defined as follows.

Pro_EC[n+1]-Pro_EC[n]/t[n+1]-t[n]: change speed of concentration of protein in second circulation flow path 62 between timing of n-th calculation and timing of (n+1)-th calculation.

L_t_w2: speed (discard speed) at which the concentration of protein changes in second circulation flow path 62 by discarding a part of culture medium via second circulation flow path 62.

E_t_m2: speed (exchange speed) at which concentration of protein changes in second circulation flow path 62 due to inflow and outflow of culture medium between inside and outside of inner pore via hollow fiber membrane 40. For this concentration, the volume of the second circulation flow path 62 is used as a denominator.

L_i_b2: speed (deterioration speed) at which concentration of protein changes in second circulation flow path 62 due to protein deterioration in second circulation flow path 62.

The acquisition unit 140 acquires Cm of expression (2) above, IC_ir of expressions (2) and (7) above, V_IC of expressions (2) and (3) above, and V_EC of expression (7) above from the information input to the input screen 146. The acquisition unit 140 acquires A_r of expression (2) above, Deg_22 of expression (2) above, k_EtoI of expression (4) above, Deg_37 of expressions (5) and (9) above, and k_ItoE of expression (8) above from the parameter information 145 stored in the second storage unit 138. The acquisition unit 140 acquires Pro_IC[n] and Pro_EC[n] from the previous calculation result. Pro_IC[0] and Pro_EC[0] are initial values of concentration of protein. The calculation result of each time is stored in the second storage unit 138. The acquisition unit 140 acquires Mx_pro[n] from the second storage unit 138. Note that, Mx_pro[n] may be zero.

The simulation execution unit 142 calculates Pro_IC[n+1] and Pro_EC[n+1] as a result of the (n+1)-th calculation by substituting each parameter acquired by the acquisition unit 140 into expressions (1) to (9) above.

The first culture form is mainly executed in the cell culture process, but may be executed in a cell collection process. In a case where the first culture form is executed in the cell collection process, for example, as illustrated in Fig. 12, the basal medium is supplied in both directions of the first inlet port 48 and the first outlet port 50 starting from the first junction 68. As a result, the cells present in the first circulation flow path 58 can be collected in the hollow fiber membrane 40. The calculation expression of the concentration of protein in the first culture form is the same in the cell culture process and the cell collection process.

[3-2-2 Calculation Method in Second Culture Form]

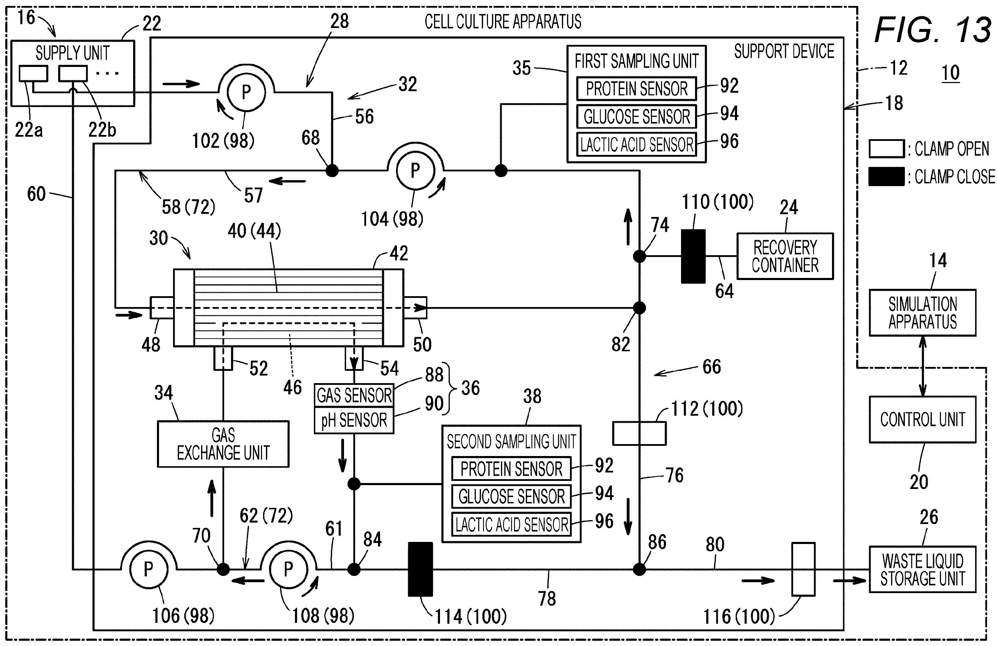

Fig. 13 is a diagram illustrating an operation of the cell culture apparatus 12 at the time of cell culture in the second culture form. The second culture form is as follows.

(a) The complete medium is supplied from the first supply unit 22a to the first circulation flow path 58.
(b) The basal medium is not supplied from the second supply unit 22b to the second circulation flow path 62.
(c) A part of the culture medium is discarded from the first circulation flow path 58 to the waste liquid storage unit 26 via the first waste liquid flow path 76.

In the second culture form, expressions (2) to (5) above, expression (8) above, and expression (9) above are used. In the second culture form, the following expressions (10) and (11) are used instead of expression (1) above. In the second culture form, the following expression (12) is used instead of expression (6) above.

[Math. 3]

$$\frac{\text{Pro\_IC}[n+1] - \text{Pro\_IC}[n]}{t[n+1] - t[n]} = \text{L\_t\_w1} + \text{F\_i\_b} + \text{L\_b\_c} + \text{E\_t\_m1} + \text{L\_i\_b1} \quad (10)$$

$$\text{L\_t\_w1} = -\frac{\text{Pro\_IC}[n] \times \text{IC\_ir}}{\text{V\_IC}} \quad (11)$$

$$\frac{\text{Pro\_EC}[n+1] - \text{Pro\_EC}[n]}{t[n+1] - t[n]} = \text{E\_t\_m2} + \text{L\_i\_b2} \quad (12)$$

A left item of expression (11) above is defined as follows.

L_t_w1: speed (discard speed) at which concentration of protein changes in first circulation flow path 58 by discarding a part of culture medium via first circulation flow path 58.

[3-2-3 Calculation Method in Third Culture Form]

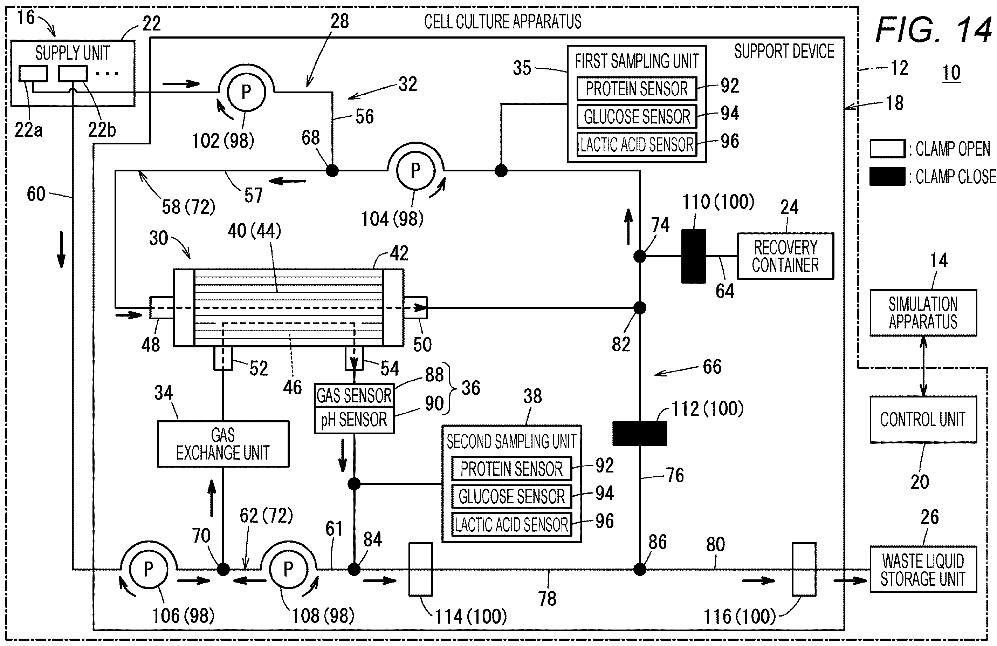

Fig. 14 is a diagram illustrating an operation of the cell culture apparatus 12 at the time of cell culture in the third culture form. The third culture form is as follows.

(a) The complete medium is supplied from the first supply unit 22a to the first circulation flow path 58.
(b) The basal medium is supplied from the second supply unit 22b to the second circulation flow path 62.
(c) A part of the culture medium is discarded from the second circulation flow path 62 to the waste liquid storage unit 26 via the second waste liquid flow path 78.

In the third culture form, expressions (1) to (6) above, expression (8) above, and expression (9) above are used. In the third culture form, the following expression (13) is used instead of expression (7) above.
[Math. 4]

$$\text{L\_t\_w2} = -\frac{\text{Pro\_EC}[n] \times (\text{IC\_ir} + \text{EC\_ir})}{\text{V\_EC}} \quad (13)$$

EC_ir included in expression (13) above is defined as follows.

EC_ir: flow rate of second supply pump 106 [unit: mL/min].

[3-2-4 Calculation Method in Fourth Culture Form]

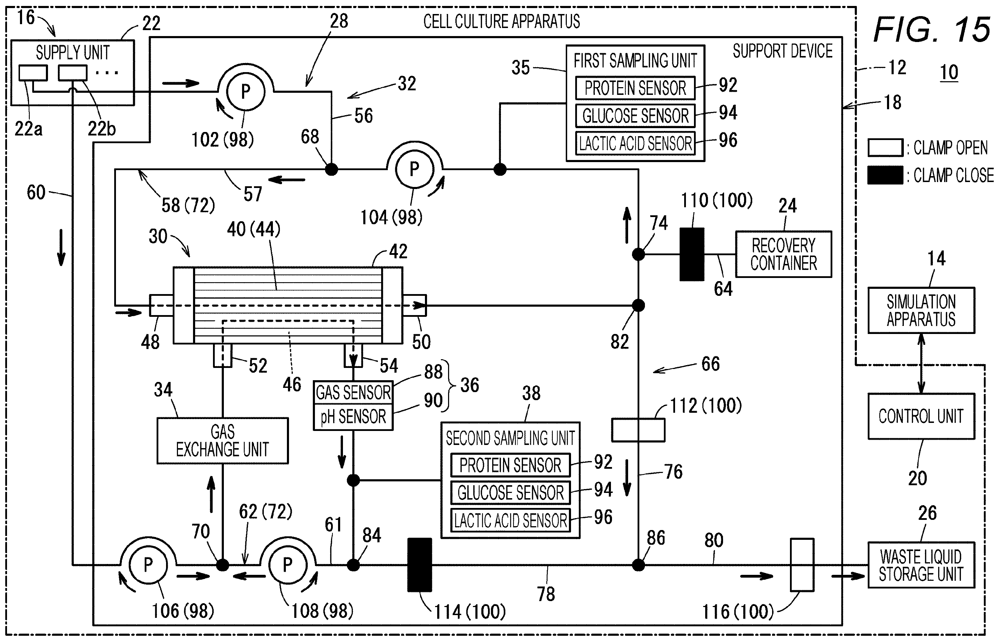

Fig. 15 is a diagram illustrating an operation of the cell culture apparatus 12 at the time of cell culture in the fourth culture form. The fourth culture form is as follows.

(a) The complete medium is supplied from the first supply unit 22a to the first circulation flow path 58.
(b) The basal medium is supplied from the second supply unit 22b to the second circulation flow path 62.
(c) A part of the culture medium is discarded from the first circulation flow path 58 to the waste liquid storage unit 26 via the first waste liquid flow path 76.

In the fourth culture form, expressions (2) to (5) above, expression (8) above, and expression (9) are used. In the fourth culture form, expression (10) above and the following expression (14) are used instead of expression (1) above. In the fourth culture form, expression (12) above is used instead of expression (6) above.
[Math. 5]

$$\text{L\_t\_w1} = -\frac{\text{Pro\_IC}[n] \times (\text{IC\_ir} + \text{EC\_ir})}{\text{V\_IC}} \quad (14)$$

[3-2-5 Calculation Method in Fifth Culture Form]

The fifth culture form is as follows. An operation of the cell culture apparatus 12 at the time of cell culture in the fifth culture form is the same as the operation of the cell culture apparatus 12 at the time of cell culture in the first culture form (Figs. 11 and 12) except for the following (a).

(a) The basal medium is supplied from the first supply unit 22a to the first circulation flow path 58.
(b) The basal medium is not supplied from the second supply unit 22b to the second circulation flow path 62.
(c) A part of the culture medium is discarded from the second circulation flow path 62 to the waste liquid storage unit 26 via the second waste liquid flow path 78.

In the fifth culture form, as in the first culture form, expressions (1) to (9) above are used. However, in the fifth culture form, since the basal medium is used instead of the complete medium, protein is not supplied from the first supply unit 22a to the first circulation flow path 58. Therefore, Cm in expression (2) above is substantially zero. That is, expression (2) above becomes the following expression (2)'.
[Math. 6]

$$\mathrm{F\_i\_b} = 0 \ (2)'$$

A main reason for supplying the basal medium to the first circulation flow path 58 is to remove waste products such as lactic acid. As cells proliferate, the waste products such as lactic acid accumulate in the first region 44 that is the inner pore of the hollow fiber membrane 40. For example, as in the second culture form, it is possible to sweep away the waste products from the inside to the outside of the first region 44 by supplying the complete medium to the first circulation flow path 58. However, when the complete medium is used for removing the waste products, protein is discarded together with the waste products. Therefore, the discarded protein is wasted. By using the basal medium instead of the complete medium, the waste products can be removed from the first region 44 while preventing the protein from being used more than necessary.
In the fifth culture form, the waste products accumulated in the first region 44 are pushed out to the second region 46 through the pore of the hollow fiber membrane 40. The waste products in the second region 46 are discarded together with the culture medium into the waste liquid storage unit 26 via the second circulation flow path 62. According to the fifth culture form, the waste products accumulated in the first region 44 can be discarded into the waste liquid storage unit 26 without significantly changing the concentration of protein in the first circulation flow path 58.
In the fifth culture form, similarly to the first culture form, the basal medium may be supplied in both directions of the first inlet port 48 and the first outlet port 50 starting from the first junction 68. As a result, the cells present in the first circulation flow path 58 can be collected in the hollow fiber membrane 40. A step in the fifth culture form is defined as the cell collection process.

[3-2-6 Calculation Method in Sixth Culture Form]

The sixth culture form is as follows. An operation of the cell culture apparatus 12 at the time of cell culture in the sixth culture form is the same as the operation of the cell culture apparatus 12 at the time of cell culture in the second culture form (Fig. 13) except for the following (a).

(a) The basal medium is supplied from the first supply unit 22a to the first circulation flow path 58.
(b) The basal medium is not supplied from the second supply unit 22b to the second circulation flow path 62.
(c) A part of the culture medium is discarded from the first circulation flow path 58 to the waste liquid storage unit 26 via the first waste liquid flow path 76.

In the sixth culture form, as in the second culture form, expressions (2) to (5) above, expression (8) above, and expressions (9) to (12) above are used. Note that, in the sixth culture form, similarly to the fifth culture form, the basal medium is used instead of the complete medium. That is, expression (2) above becomes expression (2)' above.
In the sixth culture form, the waste products and protein accumulated in the first region 44 are discarded together with the culture medium into the waste liquid storage unit 26 via the first circulation flow path 58. According to the sixth culture form, the concentration of excessive protein in the first circulation flow path 58 can be reduced, and the waste products accumulated in the first region 44 can be discarded into the waste liquid storage unit 26.

[3-2-7 Calculation Method in Seventh Culture Form]

The seventh culture form is as follows. An operation of the cell culture apparatus 12 at the time of cell culture in the seventh culture form is the same as the operation of the cell culture apparatus 12 at the time of cell culture in the third culture form (Fig. 14) except for the following (a).

(a) The basal medium is supplied from the first supply unit 22a to the first circulation flow path 58.
(b) The basal medium is supplied from the second supply unit 22b to the second circulation flow path 62.
(c) A part of the culture medium is discarded from the second circulation flow path 62 to the waste liquid storage unit 26 via the second waste liquid flow path 78.

In the seventh culture form, as in the third culture form, expressions (1) to (6) above, expression (8) above, and expressions (9) and (13) above are used. Note that, in the seventh culture form, similarly to the fifth culture form, the basal medium is used instead of the complete medium. That is, expression (2) above becomes expression (2)' above.

According to the seventh culture form, as in the fifth culture form, the waste products accumulated in the first region 44 can be discarded into the waste liquid storage unit 26 without significantly changing the concentration of protein in the first circulation flow path 58.

[3-2-8 Calculation Method in Eighth Culture Form]

The eighth culture form is as follows. An operation of the cell culture apparatus 12 at the time of cell culture in the eighth culture form is the same as the operation of the cell culture apparatus 12 at the time of cell culture in the fourth culture form (Fig. 15) except for the following (a).

(a) The basal medium is supplied from the first supply unit 22a to the first circulation flow path 58.
(b) The basal medium is supplied from the second supply unit 22b to the second circulation flow path 62.
(c) A part of the culture medium is discarded from the first circulation flow path 58 to the waste liquid storage unit 26 via the first waste liquid flow path 76.

In the eighth culture form, as in the fourth culture form, expressions (2) to (5) above, expression (8) above, expression (9) above, expression (10) above, and expressions (12) and (14) above are used. Note that, in the eighth culture form, similarly to the fifth culture form, the basal medium is used instead of the complete medium. That is, expression (2) above becomes expression (2)' above.

According to the eighth culture form, as in the sixth culture form, the concentration of excessive protein in the first circulation flow path 58 can be reduced, and the waste products accumulated in the first region 44 can be discarded into the waste liquid storage unit 26.

[3-3 Cell Culture]

Fig. 16 is a flowchart illustrating a flow of the cell culture performed using the cell culture apparatus 12. A series of steps illustrated in Fig. 16 is performed at step S5 illustrated in Fig. 10. Here, the flow of the cell culture of adherent cells will be described.

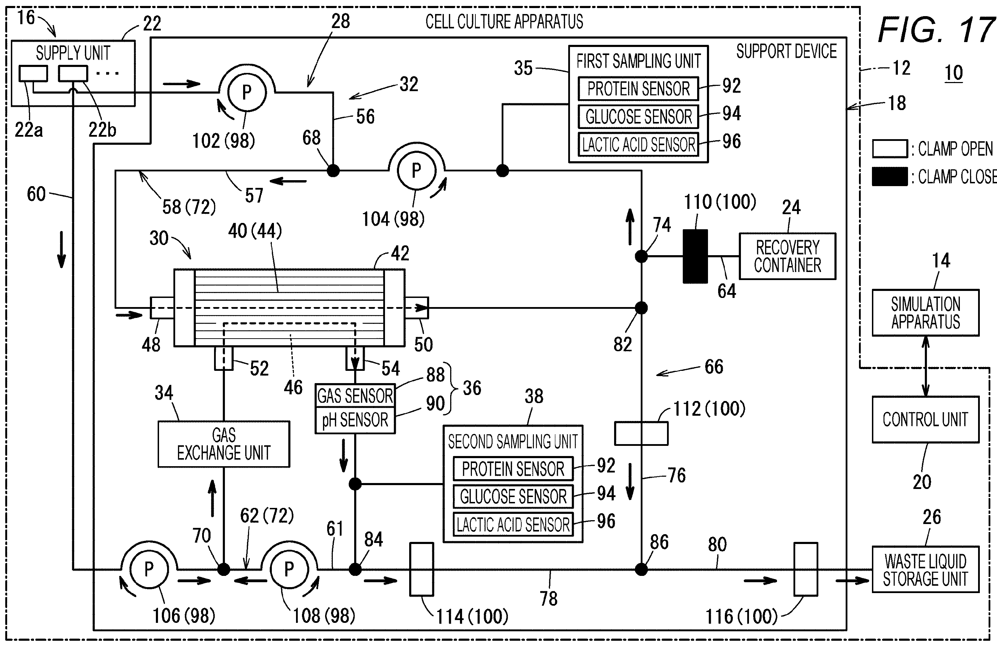

At step S11, the control unit 20 performs seeding. As illustrated in Fig. 17, the pump control unit 122 controls each pump 98. As illustrated in Fig. 17, the clamp control unit 124 controls each clamp 100. The control unit 20 controls the first supply unit 22a to supply the cell fluid to the first supply flow path 56. Then, the cell fluid is introduced from the first supply unit 22a to the first junction 68 of the first circulation flow path 58 via the first supply flow path 56. The cell fluid introduced into the first junction 68 is guided from the first inlet port 48 to the first outlet port 50 through the first region 44. In the first region 44, the cells in the cell fluid adhere to the inner surface of each hollow fiber membrane 40 of the bioreactor 30.

At step S12, the control unit 20 starts the cell culture in any one of the first to fourth culture forms. The control unit 20 can appropriately perform any one of the fifth to eighth culture forms. The control unit 20 controls the first supply unit 22a to supply the complete medium to the first supply flow path 56. Then, the complete medium is introduced from the first supply unit 22a to the first junction 68 of the first circulation flow path 58 via the first supply flow path 56. The complete medium introduced into the first junction 68 circulates in the first circulation flow path 58 including the first communication flow path 57, the first inlet port 48, the first region 44, and the first outlet port 50.

The control unit 20 controls the second supply unit 22b to supply the basal medium to the second supply flow path 60. Then, the basal medium is introduced from the second supply unit 22b to the second junction 70 of the second circulation flow path 62 via the second supply flow path 60. The basal medium introduced into the second junction 70 circulates in the second circulation flow path 62 including the second communication flow path 61, the second inlet port 52, the second region 46, and the second outlet port 54.

Furthermore, the gas exchange control unit 126 controls the gas exchange unit 34 to exchange the gas of the basal medium flowing through the second circulation flow path 62. That is, the gas exchange unit 34 allows a gas of a predetermined component to pass through the basal medium before flowing into the second inlet port 52. As a result, the gas concentration (oxygen gas concentration and carbon dioxide gas concentration) and the pH of the basal medium introduced into the second inlet port 52 of the bioreactor 30 can be adjusted to values suitable for the cell culture. In the bioreactor 30, the complete medium in the first region 44 and the basal medium in the second region 46 are exchanged via the pores of each hollow fiber membrane 40. As a result, the gas concentration and the pH of the complete medium in the first region 44 are adjusted.

The clamp control unit 124 controls the first waste liquid clamp 112 at an appropriate timing to open or close the first waste liquid flow path 76. When the first waste liquid flow path 76 is opened, a part of the complete medium in the first circulation flow path 58 is guided to the third waste liquid flow path 80 via the first waste liquid flow path 76. The clamp control unit 124 controls the second waste liquid clamp 114 at an appropriate timing to open or close the second waste liquid flow path 78. When the second waste liquid flow path 78 is opened, a part of the basal medium in the second circulation flow path 62 is guided to the third waste liquid flow path 80 via the second waste liquid flow path 78.

At step S13, the gas sensor 88 measures the oxygen concentration of the culture medium (complete medium + basal medium) and the carbon dioxide concentration of the culture medium. The pH sensor 90 measures the pH of the culture medium. The gas sensor 88 and the pH sensor 90 transmit measurement results to the control unit 20. The measurement unit 128 acquires the measurement result from each sensor. The measurement unit 128 stores the acquired measurement result in the first storage unit 120. The gas sensor 88 and the pH sensor 90 perform measurement until the cell culture ends.

At step S14, the control unit 20 samples the culture medium. The pump control unit 122 and the gas exchange control unit 126 control a pump (not illustrated) of the second sampling unit 38 and a clamp (not illustrated) of the second sampling unit 38 to sample the culture medium of the second circulation flow path 62. The sampled culture medium flows to the waste liquid storage unit 26 via each sensor of the second sampling unit 38. Here, the first sampling unit 35 may sample the basal medium.

At step S15, the glucose sensor 94 measures the glucose concentration of the culture medium. The lactic acid sensor 96 measures the lactic acid concentration of the culture medium. The glucose sensor 94 and the lactic acid sensor 96 transmit the measurement results to the control unit 20. The measurement unit 128 acquires the measurement result from each sensor. The measurement unit 128 stores the acquired measurement result in the first storage unit 120. Here, the concentration of the basal medium sampled by the first sampling unit 35 may be measured.

At step S16, the control unit 20 cleans each sensor of the second sampling unit 38. The second sampling unit 38 is provided with one or more pumps (not illustrated), one or more clamps (not illustrated), a cleaning liquid supply unit (not illustrated) and the like. The pump control unit 122 controls the pump of the second sampling unit 38. The clamp control unit 124 controls the clamp of the second sampling unit 38. The control unit 20 controls the cleaning liquid supply unit. Then, the cleaning liquid flows from the cleaning liquid supply unit to each sensor of the second sampling unit 38. As a result, each sensor of the second sampling unit 38 is cleaned. The cleaning liquid that cleans each sensor of the second sampling unit 38 flows to the waste liquid storage unit 26.

At step S17, the control unit 20 determines whether to end the cell culture on the basis of the measurement result measured by each sensor of the second sampling unit 38. In a case where the control unit 20 determines to end the cell culture (step S17: YES), the processing proceeds to step S18. In contrast, in a case where the control unit 20 determines to continue the cell culture (step S17: NO), the processing returns to step S14.

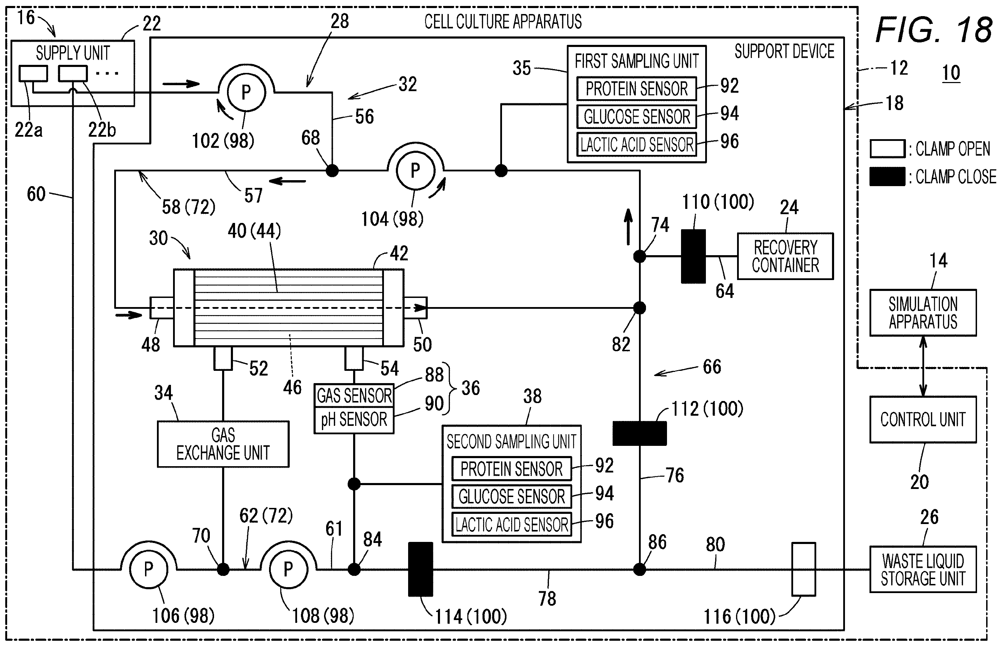

At step S18, the control unit 20 performs cell detachment. As illustrated in Fig. 18, the pump control unit 122 turns off the second supply pump 106 and the second circulation pump 108. As illustrated in Fig. 18, the clamp control unit 124 controls the first waste liquid clamp 112 and the second waste liquid clamp 114 to close the first waste liquid flow path 76 and the second waste liquid flow path 78. The control unit 20 controls the supply unit 22 to supply the detaching liquid to the first supply flow path 56. Then, the detaching liquid is guided from the supply unit 22 to the bioreactor 30 via the first supply flow path 56 and the first circulation flow path 58. In the bioreactor 30, the detaching liquid detaches the cultured cells from the inner surface of each hollow fiber membrane 40.

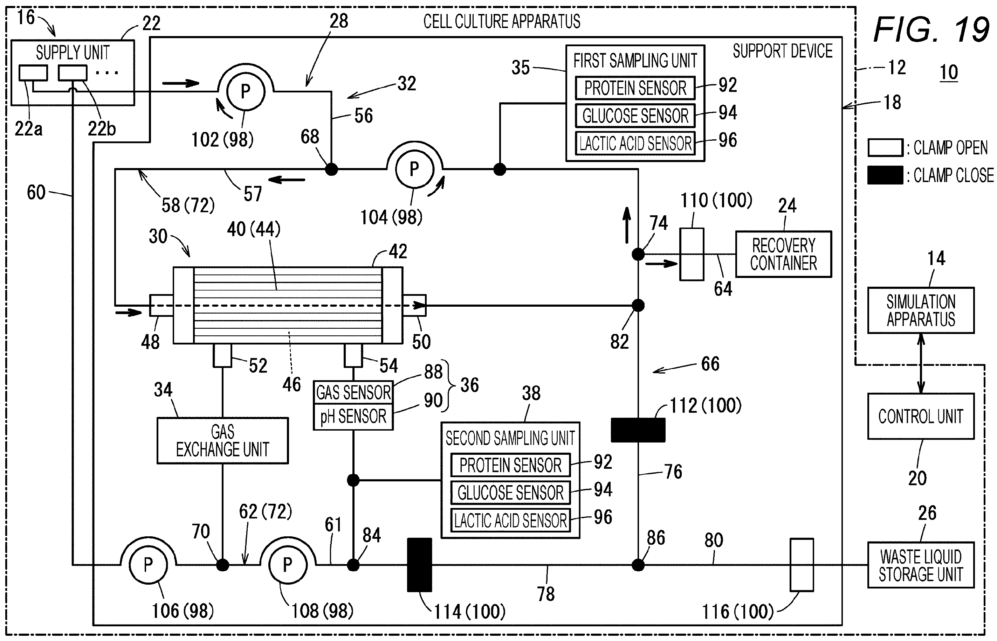

At step S19, the control unit 20 recovers the cells. As illustrated in Fig. 19, the clamp control unit 124 controls the recovery clamp 110 to open the recovery flow path 64. Then, the liquid containing the cells in the first circulation flow path 58 is guided to the recovery container 24 via the recovery flow path 64. As a result, a series of steps of the cell culture ends.

Note that, as illustrated in Fig. 10, steps S1 to S6 may be repeatedly performed. For example, steps S1 to S6 are performed N times ($N \geq 2$). At N-th step S1, the result measured in the step of the cell culture of (N-1)-th step S5 may be used as the proliferation data. In this case, the acquisition unit 140 of the simulation unit 132 acquires data of the measurement result from the first storage unit 120 of the control unit 20. Note that, each of the glucose measurement result and the lactic acid measurement result stored in the first storage unit 120 is concentration data. Each of the oxygen measurement result and the carbon dioxide measurement result stored in the first storage unit 120 is partial pressure data. In a case where the (N-1)-th measurement result is designated by the input unit 130, the acquisition unit 140 converts the concentration data and the partial pressure data into metabolic speed data. The simulation execution unit 142 simulates the cell culture using the converted data.

[4 Other Embodiments]

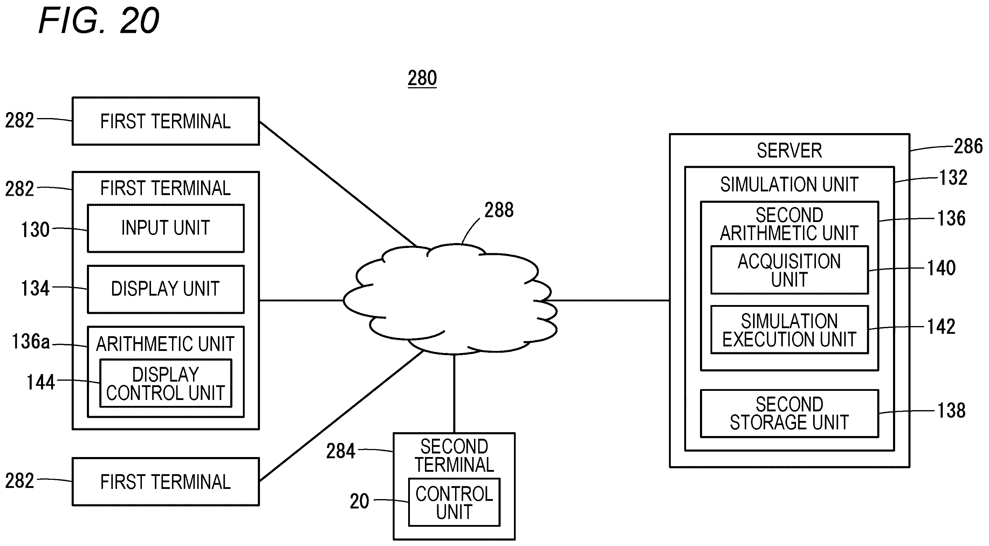

Fig. 20 is a diagram illustrating a configuration of a simulation system 280. The simulation system 280 illustrated in Fig. 20 may be used instead of the simulation apparatus 14 illustrated in Fig. 3. In Fig. 20, the same components as those in Fig. 3 are denoted by the same reference numerals. The simulation system 280 includes at least one first terminal 282, at least one second terminal 284, and a server 286.

As the first terminal 282, a personal computer, a smartphone, a tablet and the like may be used. The first terminal 282 includes the input unit 130, an arithmetic unit 136a, and the display unit 134. The arithmetic unit 136a includes a processing circuit such as a processor. The processor functions as the display control unit 144 by executing a program stored in a memory (not illustrated). The first terminal 282 is connected to a communication network 288 via a communication device not illustrated.

As the second terminal 284, a personal computer, a smartphone, a tablet and the like may be used. The second terminal 284 includes the control unit 20. The second terminal 284 is connected to the communication network 288 via a communication device not illustrated.

The server 286 includes the simulation unit 132. The server 286 is connected to the communication network 288 via a communication device not illustrated. Note that, the server 286 may be a cloud server.

The communication network 288 may be a local area network (LAN) or a wide area network (WAN). The first terminal 282, the second terminal 284, and the server 286 can communicate with one another via the communication network 288.

When the user operates the input unit 130 to input data, the first terminal 282 transmits each piece of data to the server 286. The simulation execution unit 142 of the server 286 performs simulation using the data acquired from the first terminal 282. The server 286 transmits the simulation result to the first terminal 282. The first terminal 282 acquires the simulation result from the server 286. The display control unit 144 allows the display unit 134 to display the simulation result. The second terminal 284 can acquire the data from the server 286.

[5 Operational Effects]

In the above embodiment, the simulation is executed using not only the supply speed ($F\_i\_b$) and the deterioration speeds ($L\_i\_b1$, $L\_i\_b2$) but also the exchange speeds ($E\_t\_m1$, $E\_t\_m2$). Therefore, according to the above embodiment, the accuracy of the simulation of the change in concentration of protein can be improved. The user can appropriately control an amount of protein by reflecting the simulation result in the actual culture.

In the above embodiment, the simulation is executed using the consumption speed ($L\_b\_c$). Therefore, according to the above embodiment, the accuracy of the simulation of the change in concentration of protein can be further improved.

In the above embodiment, the simulation is executed using the discard speeds ($L\_t\_w1$, $L\_t\_w2$). Therefore, according to the above embodiment, the accuracy of the simulation of the change in concentration of protein can be further improved.

[6 Example of Simulation]

The present inventors actually performed a simulation (step S2 in Fig. 10) regarding the change in concentration of protein on the basis of the above-described embodiment. Furthermore, the present inventors compared the predicted value (calculated value) obtained by the simulation with the measured value measured in the actual cell culture to evaluate the accuracy of the simulation. Note that, the concentration of protein hardly changes in the culture process of the adherent cells, but easily changes in the culture process of the floating cells. Therefore, the present inventors evaluated the accuracy of the simulation for the culture process of the floating cells. As a result, an error of the predicted value (calculated value) of the concentration of protein with respect to the measured value of the concentration of protein was within the allowable range ($\pm 5\% \times$day or less). From this, the present inventors have concluded that the simulation regarding the change in concentration of protein is appropriate. Hereinafter, an example of the simulation will be described.

The cell culture apparatus 12 used in a cell culture experiment is a Quantum cell expansion system (manufactured by Terumo BCT, Inc.) or a Quantum Flex cell expansion system (manufactured by Terumo BCT, Inc.). Hereinafter, the Quantum cell expansion system is referred to as a "first culture apparatus", and the Quantum Flex cell expansion system is referred to as a "second culture apparatus". A size of the bioreactor 30 is different between the first culture apparatus and the second culture apparatus. The size of the bioreactor 30 of the second culture apparatus is 1/10 the size of the bioreactor 30 of the first culture apparatus. Note that, at least one of the first culture apparatus and the second culture apparatus may change the size of the bioreactor 30. The hollow fiber membrane 40 provided in the culture apparatus preferably has a molecular weight cutoff of 5 to 20 kDa. The molecular weight cutoff of the hollow fiber membrane 40 of the first culture apparatus and the second culture apparatus is about 17 kDa.

[6-1 Specification of Parameter]

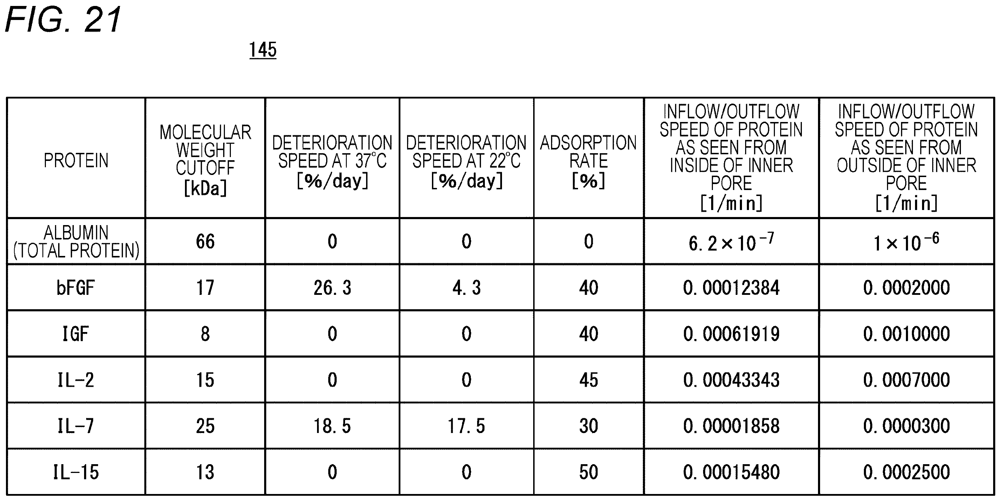

In order to perform the simulation, it is necessary to specify the parameter information 145 illustrated in Fig. 4. Therefore, the present inventors have specified numerical values of the parameter information 145 regarding several types of proteins before performing the simulation. Fig. 21 illustrates a result of specifying the numerical values of the parameter information 145. Fig. 21 is a table illustrating specific examples of the numerical values of the parameter information 145. Proteins the numerical values of which have been specified are albumin (total protein), bFGF, IGF, IL-2, IL-7, and IL-15. Hereinafter, a method of specifying the numerical value of each parameter will be described.

[6-1-1 Method of Specifying Deterioration Speed]

The present inventors placed a culture medium containing protein of a predetermined concentration in a temperature environment of 37°C, and measured the concentration of protein after a lapse of a predetermined time. The present inventors have specified the numerical value of "the deterioration speed of protein at 37°C" out of the parameter information 145 on the basis of the predetermined concentration and the measured concentration. The present inventors have specified the numerical value of "the deterioration speed of protein at 22°C" out of the parameter information 145 by the method similar to the method of specifying "the deterioration speed of protein at 37°C".

[6-1-2 Method of Specifying Inflow/Outflow Speed]

The present inventors operated the first culture apparatus so that the flow path through which the culture medium flows was the same as that in the first culture form described in [3-2-1] above. At that time, a culture medium containing protein and free from cells was set in the first supply unit 22a. The present inventors executed supply and discard of the culture medium according to the first culture form for a predetermined period. Furthermore, the present inventors periodically performed sampling in each of the first sampling unit 35 and the second sampling unit 38, and measured the concentration of protein in each culture medium. The present inventors have specified the numerical values of "inflow/outflow speed of protein as seen from inside of inner pore" and the numerical values of "inflow/outflow speed of protein as seen from outside of inner pore" on the basis of the measurement result.

[6-1-3 Method of Specifying Adsorption Rate]

The present inventors calculated the concentration of protein (Pro_IC) in the first circulation flow path 58 on the basis of expressions (1) to (5) above described in [3-2-1] above. At that time, the numerical value of "deterioration speed of protein at 37°C" specified in [6-1-1] above was substituted into Deg_37. The numerical value of "deterioration speed of protein at 22°C" specified in [6-1-1] above was substituted into Deg_22. The numerical value of "inflow/outflow speed of protein as seen from inside of inner pore" specified in [6-1-2] above was substituted into k_Etol. In contrast, each of optional numerical values not smaller than 1 (for example, 0%, 10%, ..., 90%, 100% and the like) was substituted into A_r corresponding to the adsorption rate out of the parameter information 145. Furthermore, the present inventors obtained a concentration line (referred to as a predicted concentration line) indicating the transition of the predicted value (calculated value) of the concentration of protein in a predetermined period. The predicted concentration line is obtained for each numerical value substituted into A_r. Similarly, the present inventors obtained a concentration line (referred to as a measured concentration line) indicating the transition of the measured value of the concentration of protein in a predetermined period.

Next, the present inventors compared the predicted concentration line obtained for each A_r with the measured concentration line. The present inventors selected the predicted concentration line closest to the measured concentration line, and specified A_r corresponding to the selected predicted concentration line as the "adsorption rate" included in the parameter information 145.

Each numerical value illustrated in Fig. 21 was specified by the methods [6-1-1] to [6-1-3] above. Note that, the numerical value of temperature of protein deterioration depends on a protein manufacturer. The numerical value of the adsorption rate depends on the form of the bioreactor 30 of the culture apparatus. As described above, the numerical value of the parameter information 145 changes due to various factors. That is, the numerical values illustrated in Fig. 21 are not unique values of each protein but reference values.

From the cell culture experiment and simulation to specify the parameters, the following was found. As a result, "deterioration speed" and " exchange speed" (numerical values of "inflow/outflow speed of protein as seen from inside of inner pore" and "inflow/outflow speed of protein as seen from outside of inner pore"), and "correction rate (adsorption rate)" unique to each protein could be set.

- Regarding albumin (total protein) larger than the molecular weight cutoff of the hollow fiber membrane 40, the predicted concentration line and the measured concentration line substantially coincided with each other.
- Regarding protein not larger than the molecular weight cutoff of the hollow fiber membrane 40, the concentration tended to increase in both the first circulation flow path 58 and the second circulation flow path 62, and the predicted concentration line and the measured concentration line substantially coincided with each other.
- Regarding protein other than albumin (total protein), the predicted value tended to be higher than the measured value. Assuming that the protein adsorbs in the flow path, a ratio indicating the concentration similar to that of the measured value can be calculated and used as the correction rate (adsorption rate).

[6-2 Simulation regarding Change in Protein Concentration]

After specifying the numerical values illustrated in Fig. 21, the present inventors evaluated the accuracy of simulation for each of the cell culture using the first culture apparatus and the cell culture using the second culture apparatus.

[6-2-1 Accuracy Evaluation 1]

The present inventors conducted three cell culture experiments under different culture conditions using the first culture apparatus to generate three specimens (specimen 1, specimen 2, and specimen 3) of the floating cells. In each of the three cell culture experiments, cell culture was performed for seven days, and the concentration of protein was periodically measured. Apart from this, the present inventors performed a simulation regarding the change in concentration of protein on the basis of the culture condition of each of the three specimens.
Execution conditions of the three cell culture experiments and the three simulations are described below.

- Jurkat cells were used as the floating cells to be cultured.
- Complete medium containing total protein as protein was used.
- The cell culture circuit 16 was primed with PBS, and after the priming, the bag filled with the complete medium was connected to the first supply flow path 56.
- Gas conditioning was performed, and cells were seeded in the first circulation flow path 58.
- In an initial stage of the cell culture period (seven days), a series of processes including the cell culture step, a cell dispersion step, and a cell collection step according to the first culture form were repeatedly performed. At the cell dispersion step, the supply and discard of the culture medium were stopped, and the culture medium was circulated in the first circulation flow path 58. At the cell collection step, the cells diffused into the first circulation flow path 58 at the cell dispersion step were collected inside the bioreactor 30. Here, the cell collection step was performed according to the fifth culture form.
- At the cell dispersion step, all clamps 100 (recovery clamp 110, first waste liquid clamp 112, second waste liquid clamp 114, and third waste liquid clamp 116) are closed. In this state, the concentration of protein does not change. Therefore, there is no calculation expression of the protein concentration corresponding to the cell dispersion step.
- In middle to later stages of the cell culture period (seven days), a series of processes including the cell culture step, a cell dispersion step, and a cell collection step according to the third culture form were repeatedly performed. Here, the cell collection step was performed according to the fifth culture form.

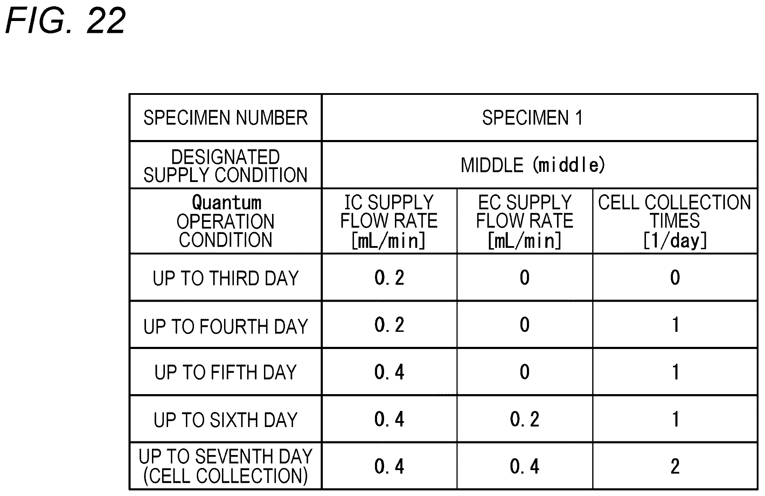

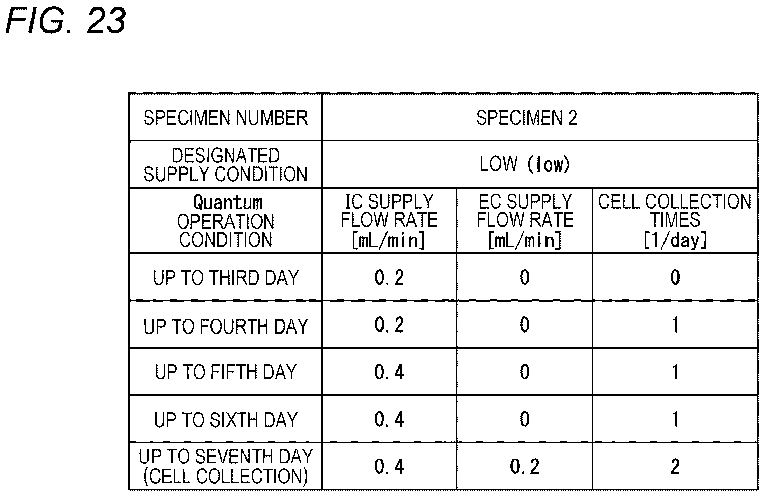

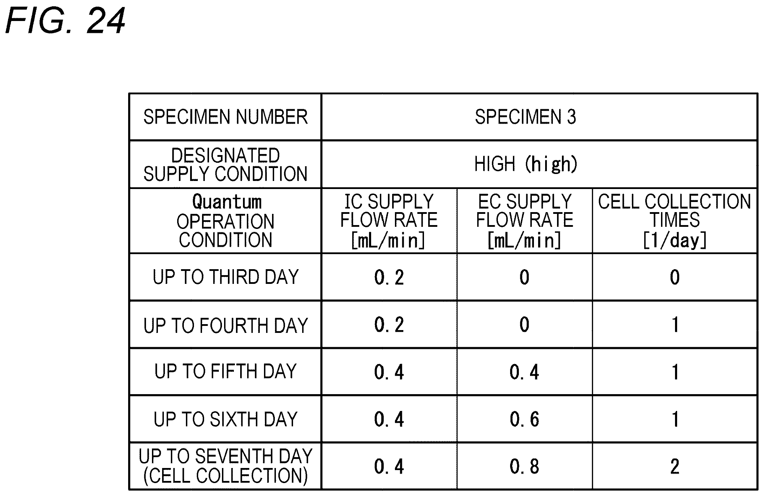

Figs. 22 to 24 are tables illustrating the culture conditions. Fig. 22 illustrates the culture condition of the specimen 1. Fig. 23 illustrates the culture condition of the specimen 2. Fig. 24 illustrates the culture condition of the specimen 3. An "IC supply flow rate" illustrated in each table is the flow rate of the complete medium supplied to the first circulation flow path 58 by the first supply pump 102. The "IC supply flow rate" corresponds to IC_ir of expression (2) above described in [3-2-1] above. An "EC supply flow rate" illustrated in each table is the flow rate of the basal medium supplied to the second circulation flow path 62 by the second supply pump 106. The "EC supply flow rate" corresponds to EC_ir of expression (13) above described in [3-2-3] above.
As illustrated in Figs. 22 to 24, in the three cell culture experiments, the flow rates of the complete medium and the basal medium and timings of switching from the first culture form to the third culture form are different from each other. Note that, in each table, a period in which the "EC supply flow rate" is zero means that the first culture form is being performed. In each Table, a period in which the "EC supply flow rate" is other than zero means that the third culture form is being performed.

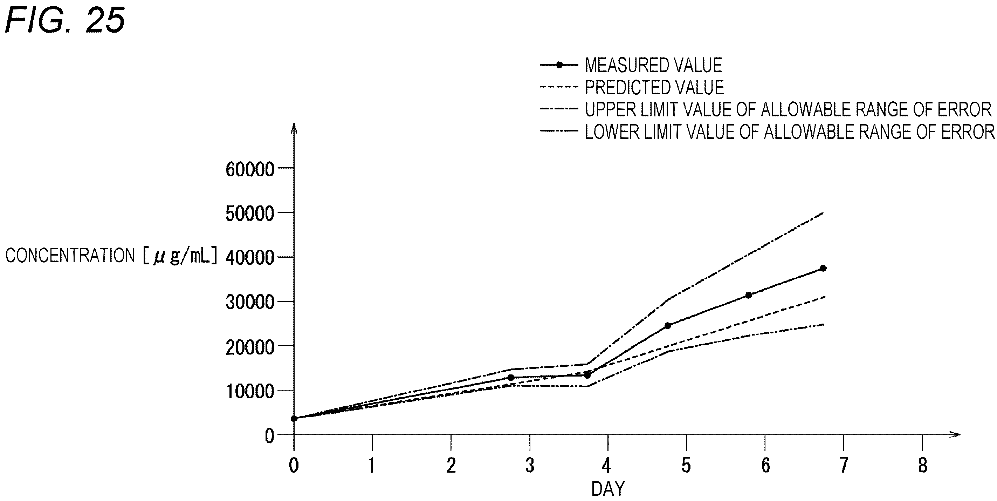

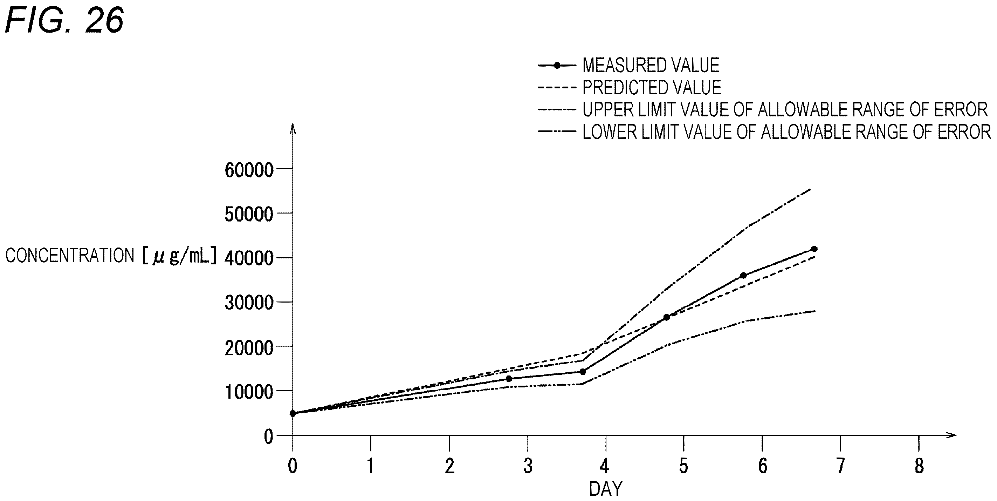

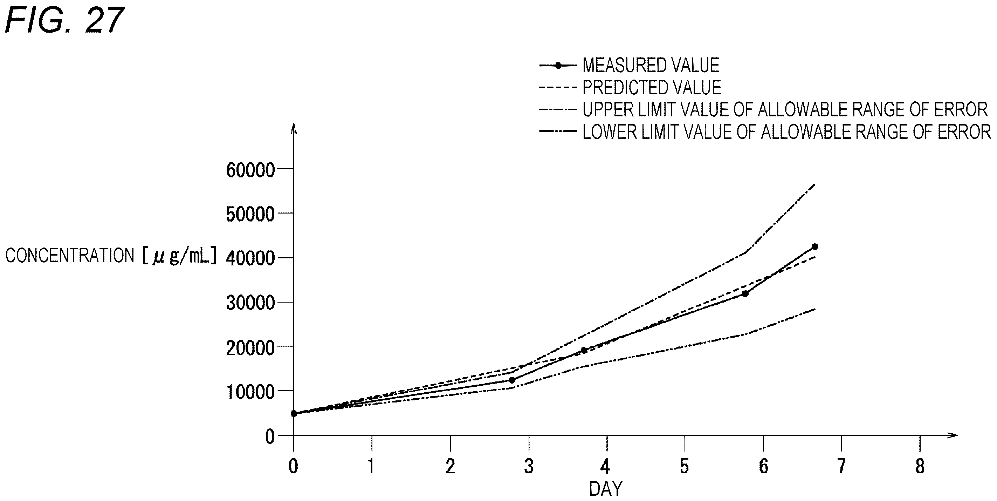

Figs. 25 to 27 are graphs illustrating the transition of the concentration of protein in the first circulation flow path 58. The graph in Fig. 25 illustrates the transition of the concentration of protein in the experiment in which the specimen 1 was cultured. The graph in Fig. 26 illustrates the transition of the concentration of protein in the experiment in which the specimen 2 was cultured. The graph in Fig. 27 illustrates the transition of the concentration of protein in the experiment in which the specimen 3 was cultured. In Figs. 25 to 27, the graph indicated by a solid line is the measured concentration line indicating the transition of the measured value of the protein (total protein) concentration. The graph indicated by a broken line is a predicted concentration line indicating the transition of the predicted value (calculated value) of the protein (total protein) concentration. The graph indicated by a dash-dot line is a line indicating an upper limit value of an allowable range of an error of the prediction value with respect to the measurement value. The graph indicated by a two-dot chain line is a line indicating a lower limit value of the allowable range of the error of the prediction value with respect to the measurement value Note that, here, the allowable range of the error is $\pm 5\% \times$day.

As illustrated in Figs. 25 to 27, each predicted concentration line for the specimens 1 to 3 generally fell within the allowable range (between the lower limit value and the upper limit value) of the error. That is, the predicted concentration line regarding each of the specimens 1 to 3 fell within the range of the measured concentration line $\pm 5\% \times$day in the middle to later stage of culture (after fourth day) in which cell proliferation was active. The predicted concentration line regarding each of the specimens 1 to 3 fell within the range of the measured concentration line $\pm 5\% \times$day on a final day of culture.

[6-2-2 Accuracy Evaluation 2]

The present inventors conducted a cell culture experiment using the second culture apparatus to generate a specimen of floating cells. In this experiment, cell culture was performed for seven days, and the concentration of protein was periodically measured. Apart from this, the present inventors performed a simulation regarding the change in concentration of protein on the basis of the culture condition of the specimen.

Execution conditions of the cell culture experiment and the simulation are described below.

- Jurkat cells were used as the floating cells to be cultured.
- Complete medium containing total protein as protein was used.
- Gas conditioning was performed, and cells were seeded in the first circulation flow path 58.
- In an entire period of the cell culture period (seven days), a series of processes including the cell culture step, a cell dispersion step, and a cell collection step according to the first culture form was repeatedly performed. Here, the cell collection step was performed according to the first culture form.

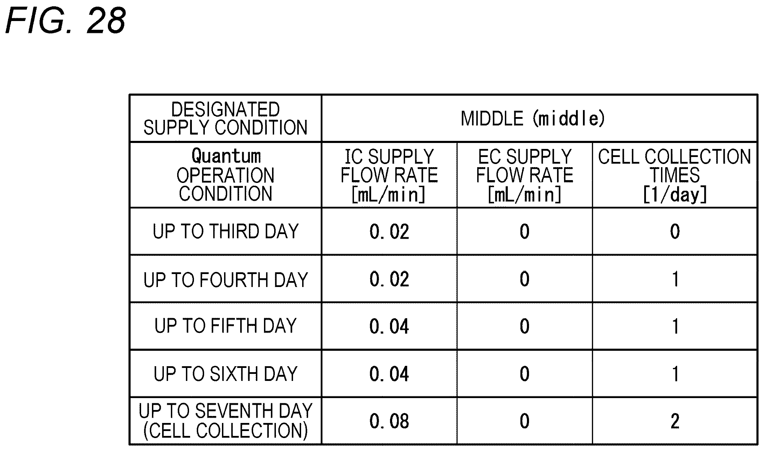

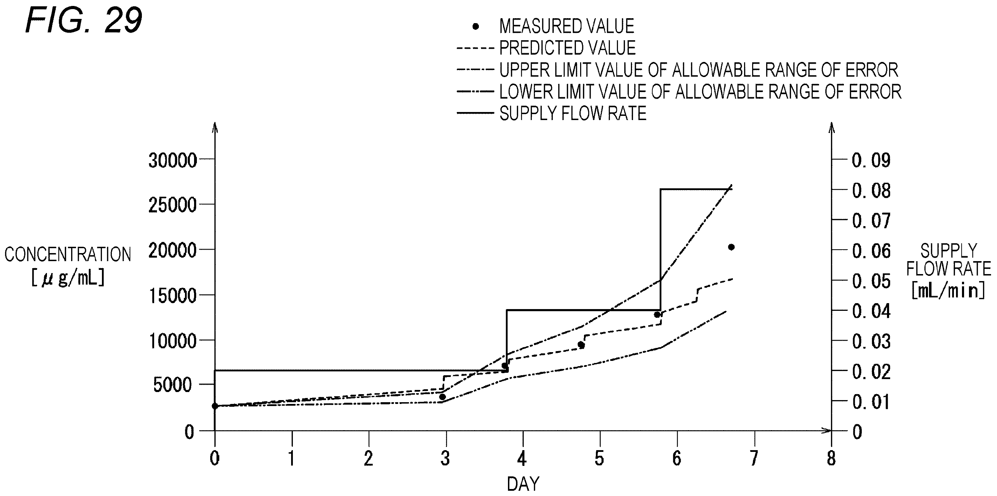

Fig. 28 is a table illustrating the culture condition. Fig. 29 is a graph illustrating the transition of the concentration of protein in the first circulation flow path 58. In Fig. 29, the graph indicated by a broken line is a predicted concentration line indicating the transition of the predicted value (calculated value) of the protein (total protein) concentration. The graph indicated by a dash-dot line is a line indicating an upper limit value of an allowable range of an error of the prediction value with respect to the measurement value. The graph indicated by a two-dot chain line is a line indicating a lower limit value of the allowable range of the error of the prediction value with respect to the measurement value Note that, here, the allowable range of the error is $\pm 5\% \times$day.

As illustrated in Fig. 29, the predicted concentration line regarding the specimen generally fell within the allowable range (between the lower limit value and the upper limit value) of the error. That is, the predicted concentration line regarding the specimen fell within the range of the measured concentration line $\pm 5\% \times$day in the middle to later (after third day) stages of culture in which cell proliferation was active. The predicted concentration line regarding the specimen fell within the range of the measured concentration line $\pm 5\% \times$day on the final day of culture.

As described above, in any of the accuracy evaluation (accuracy evaluations 1 and 2) using the two culture apparatuses having different sizes, the error of the predicted value with respect to the measured value of the concentration of protein was within the allowable range. From this, the present inventors have concluded that the simulation regarding the change in concentration of protein is appropriate.

The simulation is not affected by the culture condition. The simulation of the concentration of protein can be performed without being affected by a combination of a plurality of culture forms, switching of a plurality of culture forms, a culture scale and the like.

[6-3 Example of Cell Culture of Adherent Cells]

In the accuracy evaluations 1 and 2 described above, the measured value of the concentration of protein in the cell culture experiment of the floating cells (example) was compared with the predicted value (calculated value) of the concentration of protein in the simulation. Note that, in a case where the cell culture of the adherent cells is performed, for example, the following example can be performed.

- HEK293 cells are used as the adherent cells to be cultured.
- Complete medium containing total protein as protein is used.
- After priming the cell culture circuit 16 with PBS, the cell culture circuit 16 is precoated with cellular adhesion factors (vitronectin and fibronectin), and after the precoating, the bag filled with the complete medium is connected to the first supply flow path 56.
- Gas conditioning is performed, and cells are seeded in the first circulation flow path 58.
- In the initial stage of the cell culture period, the cell culture step according to the second culture form is performed.
- In the middle to later stages of the cell culture period, the cell culture step according to the fourth

culture form is performed.

In the second culture form and the fourth culture form, which are culture forms of the adherent cells, the complete medium is supplied to the first circulation flow path 58, and the culture medium is discarded from the first circulation flow path 58 to the waste liquid storage unit 26 via the first waste liquid flow path 76. That is, the culture medium is not discarded from the second circulation flow path 62. Therefore, the concentration of protein in the first circulation flow path 58 is substantially constant.

## Claims

1. A simulation apparatus that executes a simulation regarding cell proliferation in a cell culture apparatus that supplies a culture medium containing protein to an inner circulation path that is a circulation path including an inner pore of a cylindrical hollow fiber membrane to culture cells in the inner pore, the simulation apparatus comprising:
a simulation execution unit that executes the simulation regarding a change in concentration of the protein in the inner circulation path along with the cell proliferation under a predetermined culture condition at least using a supply speed that is a speed at which the concentration of the protein changes in the inner circulation path by supply of the culture medium to the inner circulation path, an exchange speed that is a speed at which the concentration of the protein changes in the inner circulation path by inflow and outflow of the culture medium between an inside of the inner pore and an outside of the inner pore via the hollow fiber membrane, and a deterioration speed that is a speed at which the concentration of the protein changes in the inner circulation path due to deterioration of the protein.

2. The simulation apparatus according to claim 1, further comprising:
a display control unit that allows a display unit to display information according to the concentration change obtained by the simulation.

3. The simulation apparatus according to claim 1 or 2, wherein
the simulation execution unit executes the simulation regarding the change in concentration of the protein in the inner circulation path by further using a consumption speed that is a speed at which the concentration of the protein changes in the inner circulation path by consumption of the protein by the cells.

4. The simulation apparatus according to claim 1 or 2, wherein
in a case where a part of the culture medium is discarded via the inner circulation path during cell culture, the simulation execution unit executes the simulation regarding the change in concentration of the protein in the inner circulation path by further using a discard speed that is a speed at which the concentration of the protein changes in the inner circulation path by discarding a part of the culture medium via the inner circulation path.

5. The simulation apparatus according to claim 1 or 2, wherein
the simulation execution unit executes the simulation regarding the change in concentration of the protein in the inner circulation path by further using an adsorption rate that is a ratio of the protein that cannot be consumed by the cells as the protein is adsorbed, aggregated, and deposited on at least one of an inside of the inner circulation path and an inside of an outer circulation path that is a circulation path including a region between the hollow fiber membrane and a housing that stores the hollow fiber membrane.

6. The simulation apparatus according to claim 1 or 2, wherein
the cell culture apparatus is capable of supplying a culture medium not containing the protein to an outer circulation path that is a circulation path including a region between the hollow fiber membrane and a housing that stores the hollow fiber membrane.

7. The simulation apparatus according to claim 6, wherein

in a case of culturing adherent cells, the cell culture apparatus is capable of discarding the culture medium via a waste liquid flow path connected to the inner circulation path.

8. The simulation apparatus according to claim 6, wherein
in a case of culturing floating cells, the cell culture apparatus is capable of discarding the culture medium via a waste liquid flow path connected to the outer circulation path.

9. The simulation apparatus according to claim 1 or 2, wherein

the cell culture apparatus includes an inside of the inner circulation path and an outer circulation path that is a circulation path including a region between the hollow fiber membrane and a housing that stores the hollow fiber membrane,
in a case where a part of the culture medium is discarded via the outer circulation path during cell culture, the simulation execution unit executes the simulation regarding the change in concentration of the protein in the outer circulation path along with the cell proliferation under the culture condition by using at least a discard speed that is a speed at which the concentration of the protein changes in the outer circulation path by discarding a part of the culture medium via the outer circulation path, the exchange speed, and the deterioration speed.

10. A simulation system that executes a simulation regarding cell proliferation in a cell culture apparatus that supplies a culture medium containing protein to an inner circulation path that is a circulation path including an inner pore of a cylindrical hollow fiber membrane to culture cells in the inner pore, the simulation system comprising:

a simulation execution unit that executes the simulation regarding a change in concentration of the protein in the inner circulation path along with the cell proliferation under a predetermined culture condition at least using a supply speed that is a speed at which the concentration of the protein changes in the inner circulation path by supply of the culture medium to the inner circulation path, an exchange speed that is a speed at which the concentration of the protein changes in the inner circulation path by inflow and outflow of the culture medium between an inside of the inner pore and an outside of the inner pore via the hollow fiber membrane, and a deterioration speed that is a speed at which the concentration of the protein changes in the inner circulation path due to deterioration of the protein; and
a display control unit that allows a display unit to display information according to the change in concentration obtained by the simulation.

11. A simulation method that executes a simulation regarding cell proliferation in a cell culture apparatus that supplies a culture medium containing protein to an inner circulation path that is a circulation path including an inner pore of a cylindrical hollow fiber membrane to culture cells in the inner pore, the simulation method comprising:
a simulation step of executing the simulation regarding a change in concentration of the protein in the inner circulation path along with the cell proliferation under a predetermined culture condition at least using a supply speed that is a speed at which the concentration of the protein changes in the inner circulation path by supply of the culture medium to the inner circulation path, an exchange speed that is a speed at which the concentration of the protein changes in the inner circulation path by inflow and outflow of the culture medium between an inside of the inner pore and an outside of the inner pore via the hollow fiber membrane, and a deterioration speed that is a speed at which the concentration of the protein changes in the inner circulation path due to deterioration of the protein.

12. The simulation method according to claim 11, further comprising:
a display step of allowing a display unit to display information according to the change in concentration obtained by the simulation.

13. The simulation method according to claim 11 or 12, wherein
at the simulation step, the simulation regarding the change in concentration of the protein in the inner circulation path is executed by further using a consumption speed that is a speed at which the concentration of the protein changes in the inner circulation path by consumption of the protein by the cells.

14. The simulation method according to claim 11 or 12, wherein
in a case where a part of the culture medium is discarded via the inner circulation path during cell culture, at the simulation step, the simulation regarding the change in concentration of the protein in the inner circulation path is executed by further using a discard speed that is a speed at which the concentration of the protein changes in the inner circulation path by discarding a part of the culture medium via the inner circulation path.

15. The simulation method according to claim 11 or 12, wherein
at the simulation step, the simulation regarding the change in concentration of the protein in the inner circulation path is executed by further using an adsorption rate that is a ratio of the protein that cannot be consumed by the cells as the protein is adsorbed, aggregated, and deposited on at least one of an inside of the inner circulation path and an inside of an outer circulation path that is a circulation path including a region between the hollow fiber membrane and a housing that stores the hollow fiber membrane.

16. The simulation method according to claim 11 or 12, wherein
the cell culture apparatus is capable of supplying a culture medium not containing the protein to an outer circulation path that is a circulation path including a region between the hollow fiber membrane and a housing that stores the hollow fiber membrane.

17. The simulation method according to claim 16, wherein
in a case of culturing adherent cells, the cell culture apparatus is capable of discarding the culture medium via a waste liquid flow path connected to the inner circulation path.

18. The simulation method according to claim 16, wherein
in a case of culturing floating cells, the cell culture apparatus is capable of discarding the culture medium via a waste liquid flow path connected to the outer circulation path.

19. The simulation method according to claim 11 or 12, wherein

the cell culture apparatus includes an inside of the inner circulation path and an outer circulation path that is a circulation path including a region between the hollow fiber membrane and a housing that stores the hollow fiber membrane,
in a case where a part of the culture medium is discarded via the outer circulation path during cell culture, at the simulation step, the simulation regarding the change in concentration of the protein in the outer circulation path along with the cell proliferation under the culture condition is executed by using at least a discard speed that is a speed at which the concentration of the protein changes in the outer circulation path by discarding a part of the culture medium via the outer circulation path, the exchange speed, and the deterioration speed.

*FIG. 1*

# FIG. 2

SENSOR UNIT — 36

PROTEIN SENSOR — 92

GLUCOSE SENSOR — 94

LACTIC ACID SENSOR — 96

CONTROL UNIT — 20

FIRST ARITHMETIC UNIT — 118

PUMP CONTROL UNIT — 122

CLAMP CONTROL UNIT — 124

GAS EXCHANGE CONTROL UNIT — 126

MEASUREMENT UNIT — 128

FIRST STORAGE UNIT — 120

SUPPLY UNIT — 22

GAS EXCHANGE UNIT — 34

PUMP — 98

CLAMP — 100

EP 4 722 332 A1

EP 4 722 332 A1

## FIG. 3

14

```
                          ┌─────────────────────────────┐
                          │  132                         │
                          │  SIMULATION UNIT             │
┌──────────┐              │  ┌───────────────────────┐   │      ┌──────────┐
│  INPUT   │              │  │ SECOND            136 │   │      │ DISPLAY  │
130 ─│  UNIT    │──────────────│ ARITHMETIC UNIT       │   │──────│  UNIT    │─ 134
└──────────┘              │  │  ┌─────────────────┐  │   │      └──────────┘
                          │  │  │ ACQUISITION 140 │  │   │
                          │  │  │ UNIT            │  │   │
                          │  │  └─────────────────┘  │   │
                          │  │  ┌─────────────────┐  │   │
                          │  │  │ SIMULATION  142 │  │   │
                          │  │  │ EXECUTION UNIT  │  │   │
                          │  │  └─────────────────┘  │   │
                          │  │  ┌─────────────────┐  │   │
                          │  │  │ DISPLAY     144 │  │   │
                          │  │  │ CONTROL UNIT    │  │   │
                          │  │  └─────────────────┘  │   │
                          │  └───────────────────────┘   │
                          │  ┌───────────────────────┐   │
                          │  │ SECOND          138   │   │
                          │  │ STORAGE UNIT          │   │
                          │  └───────────────────────┘   │
                          └──────────────┬──────────────┘
                                         ┆
                          ┌──────────────┴──────────────┐
                          │      CONTROL UNIT        20  │
                          └─────────────────────────────┘
```

EP 4 722 332 A1

## FIG. 4

<u>145</u>

| PROTEIN | DETERIORATION SPEED AT 37°C | DETERIORATION SPEED AT 22°C | ADSORPTION RATE | INFLOW/OUTFLOW SPEED OF PROTEIN AS SEEN FROM INSIDE OF INNER PORE | INFLOW/OUTFLOW SPEED OF PROTEIN AS SEEN FROM OUTSIDE OF INNER PORE |
|---|---|---|---|---|---|
| bFGF | X[%/day] | X[%/day] | X[%] | X[1/min] | X[1/min] |

*FIG. 5*

146

EP 4 722 332 A1

**CELL PROLIFERATION SIMULATION APPLICATION** — □ X

| Simulation parameter | Feedback condition | Cell metaborism | Graph_Main |

Scale [1.0X ▽] —154
Cell type [Default ▽] —156
Media feed type [ ▽]
Feedback [OFF ▽] —160

162 — Media(IC)
| | | |
|---|---|---|
| Glucose | XXX | mM |
| Lactate | XXX | mM |
| NaHCO₃ | XXX | mM |
| Albumin | XXX | mM |
| bFGF | XXX | mM |
| IGF | XXX | mM |
| IL-2 | XXX | mM |
| pKa | XXX | pH |
| Cost | XXX | Yen/ml |

164 — Media(EC)
| | | |
|---|---|---|
| Glucose | XXX | mM |
| Lactate | XXX | mM |
| NaHCO₃ | XXX | mM |
| pKa | XXX | pH |
| Cost | XXX | Yen/ml |

166 — Gas
| | | |
|---|---|---|
| O₂ | XXX | % |
| CO₂ | XXX | % |
| Flow rate | XXX | L/min |

168 — Other
| | | |
|---|---|---|
| IC volume | XXX | mL |
| EC volume | XXX | mL |
| Barometic P | XXX | mmHg |
| Evaporation rate | XXX | L/min |

170
| Day | IC inlet (mL/min) | IC circ (mL/min) | EC inlet (mL/min) | EC circ (mL/min) |
|---|---|---|---|---|
| 0 | XXX | XXX | XXX | XXX |
| 1 | XXX | XXX | XXX | XXX |
| 2 | XXX | XXX | XXX | XXX |
| 3 | XXX | XXX | XXX | XXX |
| 4 | XXX | XXX | XXX | XXX |
| 5 | XXX | XXX | XXX | XXX |
| 6 | XXX | XXX | XXX | XXX |
| 7 | XXX | XXX | XXX | XXX |

172    174

Days [XXX] Days
Initial cells [XXX] X 10^ [XXX] cells
Doubling time [XXX] hr
Temperature [XXX] °C

176   178

Alart range

180
| Parameter | LLR | LAR | UAR | ULR |
|---|---|---|---|---|
| Glucose | XXX | XXX | – | – |
| Lactate | – | – | XXX | XXX |
| pO₂ | XXX | XXX | – | – |
| pCO₂ | – | – | XXX | XXX |
| pH | XXX | XXX | XXX | XXX |
| Albumin | XXX | XXX | XXX | XXX |
| bFGF | XXX | XXX | XXX | XXX |
| IGF | XXX | XXX | XXX | XXX |
| IL-2 | XXX | XXX | XXX | XXX |

182 — [SAVE]

*FIG. 6*

EP 4 722 332 A1

## FIG. 7

<u>150</u>

| CELL PROLIFERATION SIMULATION APPLICATION | | | | | | | − □ ✕ |

| Simulation parameter | Feedback condition | Cell metaborism | Graph_Main | |

| No. | parameter | if | | | | Action | | | |
|---|---|---|---|---|---|---|---|---|---|
| 1 | Lactate ∨ | > ∨ | XXX | mM | | IC inlet ∨ | XXX | mL/min |
| 2 | Lactate ∨ | > ∨ | XXX | mM | | IC inlet ∨ | XXX | mL/min |
| 3 | Lactate ∨ | > ∨ | XXX | mM | | IC inlet ∨ | XXX | mL/min |
| 4 | Lactate ∨ | > ∨ | XXX | mM | | IC inlet ∨ | XXX | mL/min |
| 5 | pO2 ∨ | > ∨ | XXX | mmHg | | EC inlet ∨ | XXX | mL/min |
| 6 | pO2 ∨ | > ∨ | XXX | mmHg | | EC inlet ∨ | XXX | mL/min |
| 7 | − ∨ | > ∨ | 0 | − | | − ∨ | 0 | mL/min |
| 8 | − ∨ | > ∨ | 0 | − | | − ∨ | 0 | mL/min |
| 9 | − ∨ | > ∨ | 0 | − | | − ∨ | 0 | mL/min |
| 10 | − ∨ | > ∨ | 0 | − | | − ∨ | 0 | mL/min |
| 11 | − ∨ | > ∨ | 0 | − | | − ∨ | 0 | mL/min |
| 12 | − ∨ | > ∨ | 0 | − | | − ∨ | 0 | mL/min |
| 13 | − ∨ | > ∨ | 0 | − | | − ∨ | 0 | mL/min |
| 14 | − ∨ | > ∨ | 0 | − | | − ∨ | 0 | mL/min |
| 15 | − ∨ | > ∨ | 0 | − | | − ∨ | 0 | mL/min |
| 16 | − ∨ | > ∨ | 0 | − | | − ∨ | 0 | mL/min |

200　　　　　　　202

EP 4 722 332 A1

*FIG. 8*

152

| CELL PROLIFERATION SIMULATION APPLICATION | | | | − □ X |
|---|---|---|---|---|
| Simulation parameter | Feedback condition | Cell metaborism | Graph_Main | |

# FIG. 9

<u>152</u>

CELL PROLIFERATION SIMULATION APPLICATION   — □ X

| Simulation parameter | Feedback condition | Cell metaborism | Graph_Main |

258 — Albumin

IC   260a   260b   EC

262 — bFGF

IC   264b   264a   EC

266 — IGF

IC   268b   268a   EC

270 — IL-2

IC   272a   272b   EC

# FIG. 10

```
           ( START )
               │
               ▼◄─────────────────────┐
     ┌──────────────────────┐         │
     │      INPUT DATA       │ S1      │
     └──────────────────────┘         │
               │                       │
               ▼                       │
     ┌──────────────────────┐         │
     │  PERFORM SIMULATION   │ S2      │
     └──────────────────────┘         │
               │                       │
               ▼                       │
     ┌──────────────────────┐         │
     │ DISPLAY SIMULATION RESULT │ S3  │
     └──────────────────────┘         │
               │                       │
               ▼           S4          │
   ╱ SIMULATION NEEDS TO BE ╲──────────┤
   ╲ PERFORMED AGAIN?       ╱  YES      │
               │ NO                     │
               ▼                        │
     ┌──────────────────────┐          │
     │     CELL CULTURE      │ S5       │
     └──────────────────────┘          │
               │                        │
               ▼           S6           │
   ╱ SIMULATION NEEDS TO BE ╲───────────┘
   ╲ PERFORMED AGAIN?       ╱  YES
               │ NO
               ▼
           ( END )
```

FIG. 11

CELL CULTURE APPARATUS

SUPPORT DEVICE

□ : CLAMP OPEN
■ : CLAMP CLOSE

FIRST SAMPLING UNIT
PROTEIN SENSOR
GLUCOSE SENSOR
LACTIC ACID SENSOR

SECOND SAMPLING UNIT
PROTEIN SENSOR
GLUCOSE SENSOR
LACTIC ACID SENSOR

SUPPLY UNIT

RECOVERY CONTAINER

SIMULATION APPARATUS

CONTROL UNIT

WASTE LIQUID STORAGE UNIT

GAS EXCHANGE UNIT

GAS SENSOR
pH SENSOR

EP 4 722 332 A1

FIG. 12

CELL CULTURE APPARATUS

SUPPORT DEVICE

□ : CLAMP OPEN

■ : CLAMP CLOSE

16 22 28

SUPPLY UNIT

22a 22b

35 FIRST SAMPLING UNIT

PROTEIN SENSOR 92

GLUCOSE SENSOR 94

LACTIC ACID SENSOR 96

102 (98) 68 56 32

57

58 (72) 104 (98)

60

110 (100) 24

74 RECOVERY CONTAINER

64

30 40 (44) 42

48 50 82

52 46 54 88 90 36 66

34

GAS SENSOR 38

pH SENSOR

GAS EXCHANGE UNIT

SECOND SAMPLING UNIT

112 (100)

PROTEIN SENSOR 92

GLUCOSE SENSOR 94

LACTIC ACID SENSOR 96

76

70 62 (72) 61 84 86 80

106 (98) 108 (98) 78

114 (100) 116 (100)

12 10

18

14 SIMULATION APPARATUS

CONTROL UNIT

20

26 WASTE LIQUID STORAGE UNIT

EP 4 722 332 A1

FIG. 13

FIG. 14

EP 4 722 332 A1

FIG. 15

# FIG. 16

CELL CULTURE

( START )

| | |
|---|---|
| SEEDING | S11 |

| | |
|---|---|
| START CELL CULTURE | S12 |

| | |
|---|---|
| SENSOR MEASUREMENT | S13 |

| | |
|---|---|
| SAMPLING | S14 |

| | |
|---|---|
| BIOSENSOR MEASUREMENT | S15 |

| | |
|---|---|
| CLEAN BIOSENSOR | S16 |

END CELL CULTURE?  S17  NO

YES

| | |
|---|---|
| CELL RELEASE | S18 |

| | |
|---|---|
| CELL RECOVERY | S19 |

( END )

FIG. 17

EP 4 722 332 A1

FIG. 18

FIG. 19

## FIG. 20

282 — FIRST TERMINAL

280

282 — FIRST TERMINAL
- 130 — INPUT UNIT
- 134 — DISPLAY UNIT
- 136a — ARITHMETIC UNIT
  - 144 — DISPLAY CONTROL UNIT

282 — FIRST TERMINAL

288

284 — SECOND TERMINAL
- 20 — CONTROL UNIT

SERVER — 286
- SIMULATION UNIT — 132
  - SECOND ARITHMETIC UNIT — 136
    - ACQUISITION UNIT — 140
    - SIMULATION EXECUTION UNIT — 142
  - SECOND STORAGE UNIT — 138

EP 4 722 332 A1

## FIG. 21

145

| PROTEIN | MOLECULAR WEIGHT CUTOFF [kDa] | DETERIORATION SPEED AT 37°C [%/day] | DETERIORATION SPEED AT 22°C [%/day] | ADSORPTION RATE [%] | INFLOW/OUTFLOW SPEED OF PROTEIN AS SEEN FROM INSIDE OF INNER PORE [1/min] | INFLOW/OUTFLOW SPEED OF PROTEIN AS SEEN FROM OUTSIDE OF INNER PORE [1/min] |
|---|---|---|---|---|---|---|
| ALBUMIN (TOTAL PROTEIN) | 66 | 0 | 0 | 0 | $6.2 \times 10^{-7}$ | $1 \times 10^{-6}$ |
| bFGF | 17 | 26.3 | 4.3 | 40 | 0.00012384 | 0.0002000 |
| IGF | 8 | 0 | 0 | 40 | 0.00061919 | 0.0010000 |
| IL-2 | 15 | 0 | 0 | 45 | 0.00043343 | 0.0007000 |
| IL-7 | 25 | 18.5 | 17.5 | 30 | 0.00001858 | 0.0000300 |
| IL-15 | 13 | 0 | 0 | 50 | 0.00015480 | 0.0002500 |

EP 4 722 332 A1

# FIG. 22

| SPECIMEN NUMBER | SPECIMEN 1 | | |
|---|---|---|---|
| DESIGNATED SUPPLY CONDITION | MIDDLE (middle) | | |
| Quantum OPERATION CONDITION | IC SUPPLY FLOW RATE [mL/min] | EC SUPPLY FLOW RATE [mL/min] | CELL COLLECTION TIMES [1/day] |
| UP TO THIRD DAY | 0.2 | 0 | 0 |
| UP TO FOURTH DAY | 0.2 | 0 | 1 |
| UP TO FIFTH DAY | 0.4 | 0 | 1 |
| UP TO SIXTH DAY | 0.4 | 0.2 | 1 |
| UP TO SEVENTH DAY (CELL COLLECTION) | 0.4 | 0.4 | 2 |

EP 4 722 332 A1

# FIG. 23

| SPECIMEN NUMBER | SPECIMEN 2 | | |
|---|---|---|---|
| DESIGNATED SUPPLY CONDITION | LOW (low) | | |
| Quantum OPERATION CONDITION | IC SUPPLY FLOW RATE [mL/min] | EC SUPPLY FLOW RATE [mL/min] | CELL COLLECTION TIMES [1/day] |
| UP TO THIRD DAY | 0.2 | 0 | 0 |
| UP TO FOURTH DAY | 0.2 | 0 | 1 |
| UP TO FIFTH DAY | 0.4 | 0 | 1 |
| UP TO SIXTH DAY | 0.4 | 0 | 1 |
| UP TO SEVENTH DAY (CELL COLLECTION) | 0.4 | 0.2 | 2 |

EP 4 722 332 A1

## FIG. 24

| SPECIMEN NUMBER | SPECIMEN 3 | | |
|---|---|---|---|
| DESIGNATED SUPPLY CONDITION | HIGH (high) | | |
| Quantum OPERATION CONDITION | IC SUPPLY FLOW RATE [mL/min] | EC SUPPLY FLOW RATE [mL/min] | CELL COLLECTION TIMES [1/day] |
| UP TO THIRD DAY | 0.2 | 0 | 0 |
| UP TO FOURTH DAY | 0.2 | 0 | 1 |
| UP TO FIFTH DAY | 0.4 | 0.4 | 1 |
| UP TO SIXTH DAY | 0.4 | 0.6 | 1 |
| UP TO SEVENTH DAY (CELL COLLECTION) | 0.4 | 0.8 | 2 |

# FIG. 25

Legend:
- ●— MEASURED VALUE
- ---- PREDICTED VALUE
- —··— UPPER LIMIT VALUE OF ALLOWABLE RANGE OF ERROR
- —··— LOWER LIMIT VALUE OF ALLOWABLE RANGE OF ERROR

Y-axis: CONCENTRATION [μg/mL]

X-axis: DAY

## FIG. 26

Legend:
- ● — MEASURED VALUE
- ----- PREDICTED VALUE
- —·— UPPER LIMIT VALUE OF ALLOWABLE RANGE OF ERROR
- —··— LOWER LIMIT VALUE OF ALLOWABLE RANGE OF ERROR

CONCENTRATION [μg/mL] (y-axis: 0, 10000, 20000, 30000, 40000, 50000, 60000)

DAY (x-axis: 0, 1, 2, 3, 4, 5, 6, 7, 8)

*FIG. 27*

# FIG. 28

| DESIGNATED SUPPLY CONDITION | MIDDLE (middle) | | |
|---|---|---|---|
| Quantum OPERATION CONDITION | IC SUPPLY FLOW RATE [mL/min] | EC SUPPLY FLOW RATE [mL/min] | CELL COLLECTION TIMES [1/day] |
| UP TO THIRD DAY | 0.02 | 0 | 0 |
| UP TO FOURTH DAY | 0.02 | 0 | 1 |
| UP TO FIFTH DAY | 0.04 | 0 | 1 |
| UP TO SIXTH DAY | 0.04 | 0 | 1 |
| UP TO SEVENTH DAY (CELL COLLECTION) | 0.08 | 0 | 2 |

EP 4 722 332 A1

FIG. 29

• MEASURED VALUE
---- PREDICTED VALUE
—·— UPPER LIMIT VALUE OF ALLOWABLE RANGE OF ERROR
—··— LOWER LIMIT VALUE OF ALLOWABLE RANGE OF ERROR
—— SUPPLY FLOW RATE

## INTERNATIONAL SEARCH REPORT

| International application No. |
|---|
| **PCT/JP2024/021739** |

### A. CLASSIFICATION OF SUBJECT MATTER

*C12M 1/00*(2006.01)i; *C12M 1/34*(2006.01)i; *C12N 5/00*(2006.01)i
FI: C12M1/00 C; C12M1/34 A; C12N5/00

According to International Patent Classification (IPC) or to both national classification and IPC

### B. FIELDS SEARCHED

Minimum documentation searched (classification system followed by classification symbols)

C12M1/00-3/10; C12N1/00-7/08; C12Q1/00-3/00; C07K1/00-19/00; B01D53/22; B01D61/00-71/82; C02F1/44; C02F3/28-3/34

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII)

### C. DOCUMENTS CONSIDERED TO BE RELEVANT

| Category* | Citation of document, with indication, where appropriate, of the relevant passages | Relevant to claim No. |
|---|---|---|
| A | JP 2021-048773 A (TERUMO KABUSHIKI KAISHA) 01 April 2021 (2021-04-01) | 1–19 |
| A | MENSHUTINA, Natalia V. et al., "Modelling of hollow fiber membrane bioreactor for mammalian cell cultivation using computational hydrodynamics", Bioprocess and Biosystems Engineering, 30 November 2019, vol. 43, no. 3, pp. 549-567, DOI: 10.1007/s00449-019-02249-9 | 1-19 |
| A | JP 2021-522826 A (HUACELLS CORP.) 02 September 2021 (2021-09-02) | 1-19 |
| A | JP 2017-509344 A (TERUMO BCT INC.) 06 April 2017 (2017-04-06) | 1–19 |
| A | US 2017/0147742 A1 (NANYANG TECHNOLOGICAL UNIVERSITY) 25 May 2017 (2017-05-25) | 1-19 |

☐ Further documents are listed in the continuation of Box C.     ☑ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **05 August 2024** | **20 August 2024** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (July 2022)

## INTERNATIONAL SEARCH REPORT
### Information on patent family members

International application No.

**PCT/JP2024/021739**

| Patent document cited in search report | | | Publication date (day/month/year) | Patent family member(s) | | | Publication date (day/month/year) |
|---|---|---|---|---|---|---|---|
| JP | 2021-048773 | A | 01 April 2021 | (Family: none) | | | |
| JP | 2021-522826 | A | 02 September 2021 | US | 2019/0352589 | A1 | |
| | | | | WO | 2019/219050 | A1 | |
| | | | | EP | 3775156 | A1 | |
| | | | | CN | 110499252 | A | |
| JP | 2017-509344 | A | 06 April 2017 | US | 2015/0275170 | A1 | |
| | | | | WO | 2015/148704 | A1 | |
| | | | | EP | 3122866 | A1 | |
| | | | | CN | 106232800 | A | |
| US | 2017/0147742 | A1 | 25 May 2017 | WO | 2015/199614 | A1 | |
| | | | | EP | 3161697 | A1 | |
| | | | | CN | 106663146 | A | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2020171241 A

### Non-patent literature cited in the description

- EXPERIMENTAL DETERMINATION OF THE VOLUMETRIC MASS TRANSFER COEFFICIENT. *Journal of Chemical Technology and Metallurgy*, 2013, vol. 48 (4), 351-356